# EUROPEAN PATENT APPLICATION

(11) **EP 2 371 817 A1**
(43) Date of publication of application: **05.10.2011**
(21) Application number: 10159019.8
(22) Date of filing: 01.04.2010
(51) Int. Cl.: C07D 211/46

(54) **Process for the preparation of 1-[4-(1,1-dimethylethyl)phenyl]-4-[4-(diphenylmethoxy)-1-piperidinyl]-1-butanone and acid addition salts thereof**

(71) Applicant: Arevipharma GmbH, 01445 Radebeul (DE)
(72) Inventor: Schickaneder, Christian, 01309 Dresden (DE); McGrath, Matthew, 68623 Lampertheim (DE); Schäfer, Jürgen, 01445 Radebeul (DE); Schubert, Jana, 01640 Coswig (DE); Jacob, Ulrike, 01445 Radebeul (DE); Märtens, Welljanne, 01127 Dresden (DE)
(74) Representative: Prüfer & Partner GbR European Patent Attorneys

(57) **Abstract**

The present invention describes processes for the preparation of 1-[4-(1,1-dimethylethyl)phenyl]-4-[4-(diphenylmethoxy)-1-piperidinyl]-1-butanone and acid addition salts thereof.

## Description

### Field of the invention

The present invention relates in general to the field of organic chemistry and in particular to the preparation of acid addition salts of amino ketones of formula **I** that can be transformed into pharmaceutically pure non-sedating H₁ antihistamines, in particular to a compound of formula I" defined by the following formula wherein Z is benzhydryloxy, Y is hydrogen and R¹ is methyl.

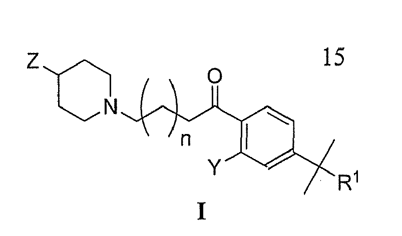

### Background of the invention

Previously described syntheses of compounds of formula I typically rely on the reaction between a piperidino derivative of formula **II** 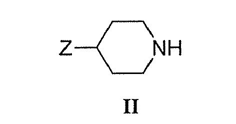 , wherein Z represents hydroxyl, benzhydryloxy, or diaryl carbinol, and a chloroketone of formula **III** 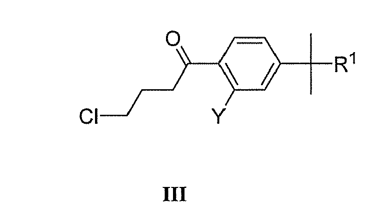 , wherein Y represents hydrogen or alkoxy and R¹ represents alkyl or carboxy, respectively, or chloroketals of formula **IV** 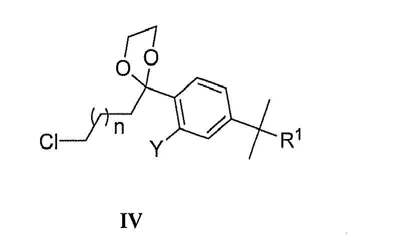 , whereas R¹ and Y are defined as above and n is 1 or 2. Alternatively, the benzhydryl ether moiety of compounds of formula **I'** 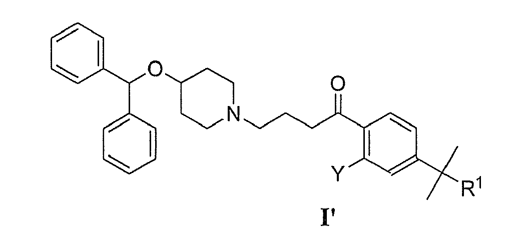 , wherein Y and R¹ are as defined above may be prepared from benzhydryl halides of formula **V** 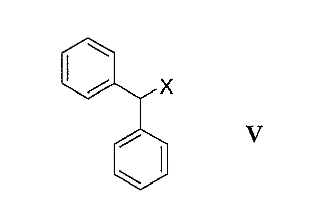 , wherein X represents halogen, and in particular bromide and chloride that are considered as potential genotoxic substances, and hydroxyl piperdino compounds of formula **VI** 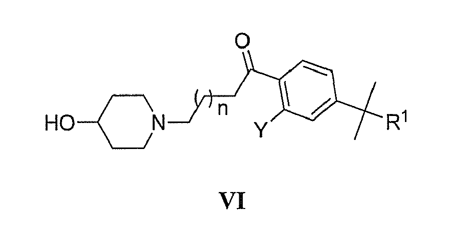 , wherein R¹, Y, and n are as defined above.

Although no examples of compounds of formula I" 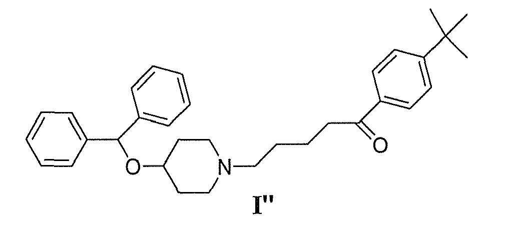 are given in EP877733B1, this patent deals with the preparation of closely related compounds of general formula **I** by reaction of compounds of formula **II**, wherein Z represents benzhydryl ether or a diaryl carbinol, and chlorobutyrophenones of general formula **III** , wherein Y is hydrogen and R¹ represents alkyl or hydrogen in the presence of a suitable base such as sodium hydrogen carbonate or sodium carbonate and optionally a catalytic amount of potassium iodide in a suitable solvent such as methanol, methyl isobutyl ketone, DMF, or toluene at a temperature between 25 °C and 150 °C, for a period of 4 to 120 h. For example, according to the procedures reported therein compounds of formula **I**"' are accessible by reaction of a compound of formula **II**' with a compound of formula **III**' (Scheme 1).

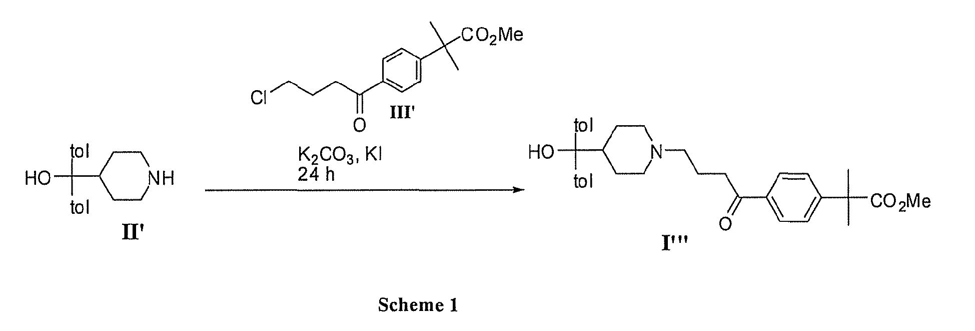

The authors exclusively report synthesis with compounds of general formula **III**, wherein R¹ is carboxo and Y is hydrogen. Also acid addition salts (including phosphate and sulfonate salts) of a compound of formula **I**', wherein Y represents hydrogen and R¹ is carboxylate are claimed therein.

For example, EP0134124A1 discloses the preparation of fumarate salts of a compound of formula **I**" but they were not used to produce the free base as a pure substance. Two synthetic strategies were employed. As shown in Scheme 2, a compound of formula **II**" was alkylated with a compound of formula **III**" in methyl isobutyl ketone and sodium carbonate. Then, the reaction product was isolated as the fumarate salt.

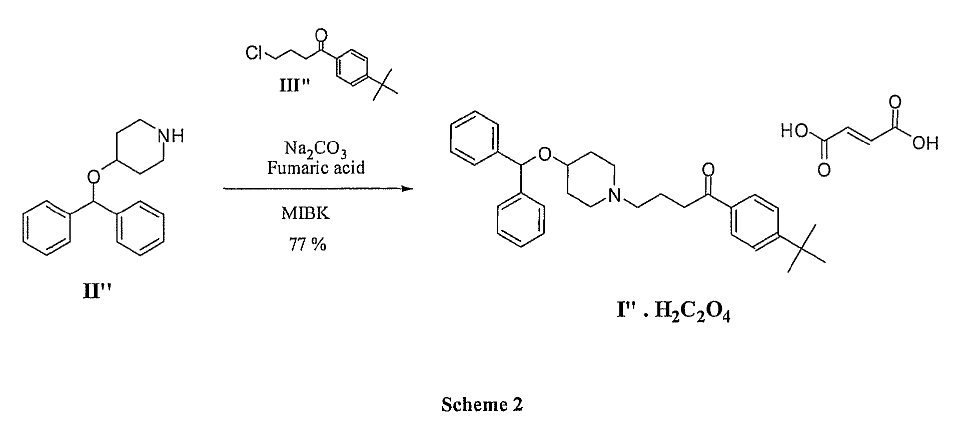

According to Scheme 3, 4-hydroxypiperidine derivative **VI**' is reacted with benzhydryl bromide of formula **V**' in methyl isobutyl ketone in the presence of sodium carbonate, followed by the precipitation of the corresponding fumarate salt of a compound of formula **I**" in 70 % yield. 4-Hydroxypiperidine derivative **VI**', in turn, was obtained by reacting 4-hydroxypiperidine with chloro ketone **III**" by using sodium hydrogen carbonate and a crystal of iodine in toluene at reflux temperature. Crude material of formula **VI**' was isolated as its fumaric acid addition salt and subsequently neutralized to give compound of formula **VI**' in 84 % yield from 4-hydroxypiperidine. However, also in this case a compound of formula **I**" was not prepared in its free base form.

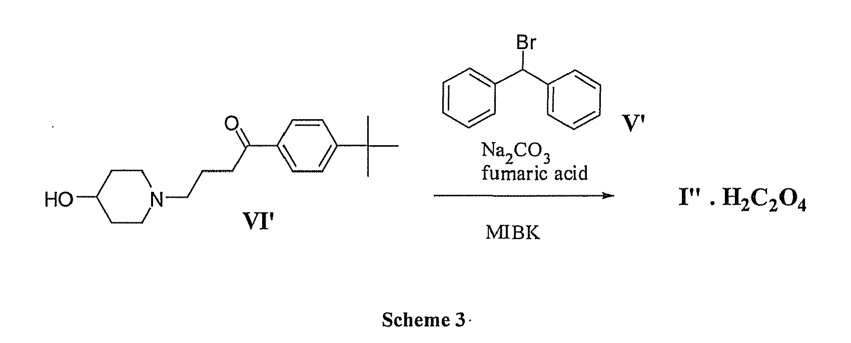

WO2009/157006 discloses a process comprising reacting a compound of formula **III** (R¹ being methyl) with hydroxypyridine (compound **II** with Z being OH) and subsequently reacting the obtained product with diphenyl methanol (compound **V** with X being OH) to obtain fumarate salt I".C₄H₄O_{4.} The ebastine fumarate salt is isolated and treated using a base to yield free base I".

CN1887866 provides a method for significantly accelerating the conversion according to Scheme 4 by utilizing phenyl acetyl peroxide in catalytic amounts as an initiator. However, the protection of the reaction mixture from oxidation is an often-neglected issue in the synthesis of compounds of type **I** as coloured impurities are formed that result in discoloured products. In light of this it is questionable whether this embodiment of the proposed process is beneficial with respect to the purity of the final product. The reported process in turn discloses a purification protocol for the compound of formula **I"** that involves an acidic extraction of compound **I"** to the aqueous phase followed by neutralization with aqueous sodium hydroxide.

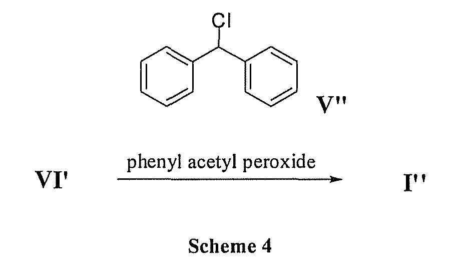

However, both EP0134124A1 and CN1887866 use the strong alkylating agents benzhydryl bromide (**V'**) and chloride (**V"**) respectively in the last step, which are considered as potentially genotoxic substances.

Indian Appl. No. 738/MUM/2006 (published 22.08.2006, Journal No. 34/2008) describes a specialized recrystallization protocol for a compound of formula **I"**, which yields crystals from an alcoholic solvent that comprise a specific particle size distribution (PSD). This procedure is directed to PSD requirements only and does not provide an improvement for the purification of the final product. Moreover the protocol requires a very specific temperature regime and a specific solvent/solute ratio. Additionally, activated charcoal was used to clarify the reaction solution although in our hands use of charcoal led to discolouration of the product when the recrystallization was attempted from ethanol.

DE3005948 discloses the synthesis of compounds of formula **I,** such as **I** wherein Z represents Ph₂COH, Y is hydrogen, and R¹ represents COOH from piperidino derivatives of formula **II** wherein Z represents Ph₂COH and a chloroketone of formula **III,** wherein R¹ represents COOH, and Y is hydrogen. An example of **I"**. HCl synthesis is not directly disclosed, however for the related compounds **II** wherein Z is CPh₂OH and **III** wherein R represents COOH, and Y is hydrogen the reaction time was too long (72 h), even in the presence of a catalytic quantity of potassium iodide (Scheme 5) and repeated recrystallization from methanol/butanone and methanol was required to afford a product of acceptable purity.

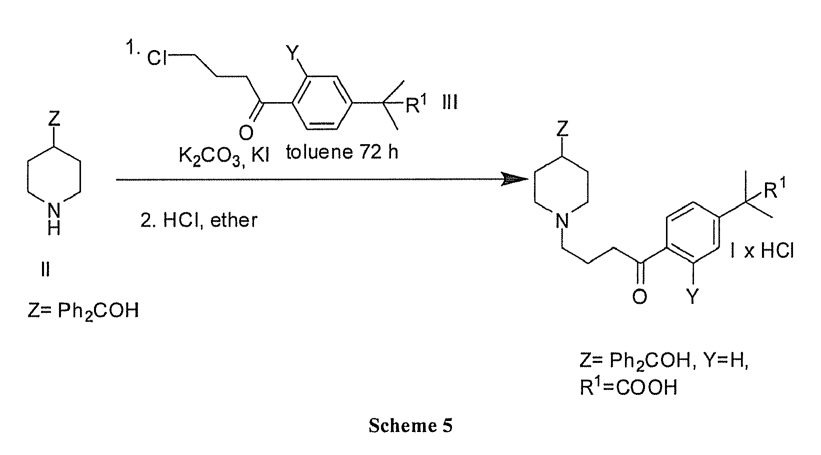

ES8606856A1 discloses the use of a chloro ketal intermediate **IV'** for the synthesis of compound **I** - HCl, wherein Z is Ph₂COH, Y is hydrogen, R¹ is methyl by reaction with a compound of formula **II** wherein Z is CO₂Et and subsequent deprotection. The chloroketone **III"** was protected as a cyclic ketal **IV'** and was then reacted with a 4-substituted piperidine derivative in acetonitrile, in the presence of tetrabutylammonium bromide and potassium carbonate. The ester function at the 4-position of the piperidine ring was converted to the tertiary alcohol with phenylmagnesium bromide and subsequent acidic deprotection affords the salt **I** . HCl (Z=Ph₂COH, Y=H, R¹=CH₃). This sequence was not applied to the synthesis of **I"** and the use of protecting groups introduces unnecessary extra steps leading to a long, complex synthesis. Compound **I**· HCl (Z=Ph₂COH, Y=H, R¹=CH₃) was neutralized with hydroxide to afford a toluene solution of **I**, which was used in the synthesis of an amino alcohol. However the free base was not isolated as such.

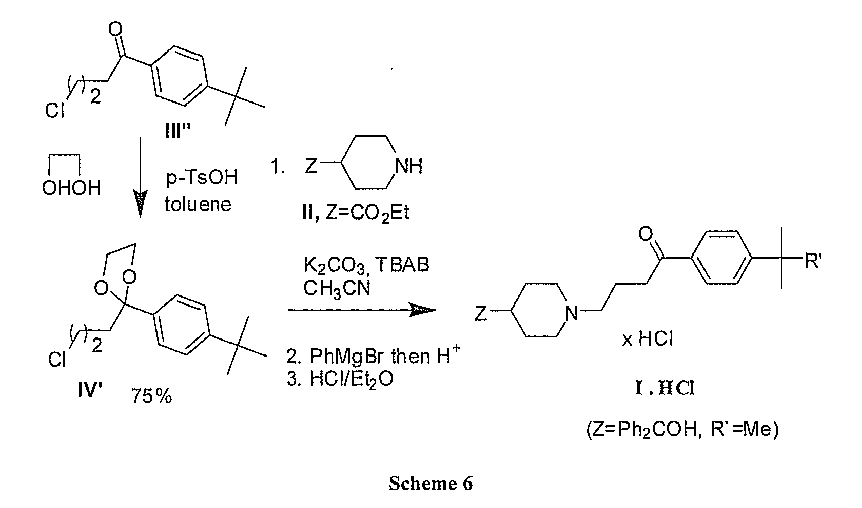

The object of the present invention is to provide an improved process for the preparation of pure 1-[4-(1,1-dimethylethyl)phenyl]-4-[4-(diphenylmethoxy)-1-piperidinyl]-1-butanone compounds of formula **I"**, and to provide useful preparative intermediates for obtaining pure compounds of formula **I"** and isolated acid addition salts of a compound of formula **I"** as important preparative intermediates.

### Summary of the invention

Various aspects, advantageous features and preferred embodiments of the present invention as summarized in the following items, respectively alone or in combination, contribute to solving the object of the invention:
(1) A process to prepare a compound of formula **I"** in free base form or in salt form, comprising: 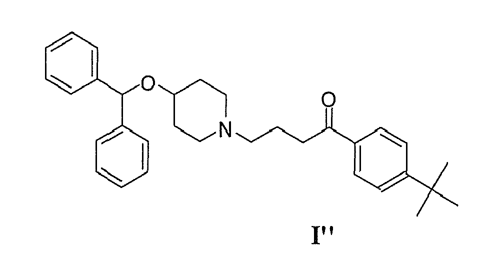
(i) reacting a compound of formula V"' 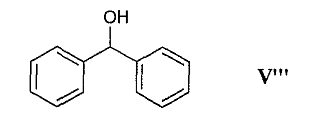 with 4-hydroxypiperidine or an acid addition salt thereof in the presence of a solvent or mixture of solvents to obtain a compound of formula **II"** 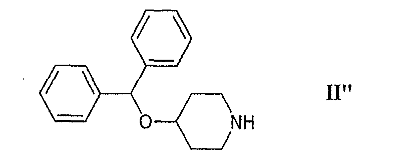 or an acid addition salt thereof,
(ii) subsequently reacting the compound of formula **II"** or the acid addition salt thereof with a compound of formula **III"'** 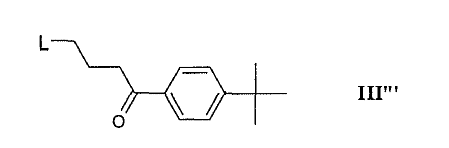 , wherein L represents a leaving group, in the presence of a solvent or mixture of solvents, preferably in the presence of an auxiliary base, to produce a compound of formula **I"** or an acid addition salt thereof; and
(iii) isolating and optionally recrystallizing the free base of the compound of formula **I"**,
   and/or
(iii') precipitating or crystallizing an acid addition salt of the compound of formula **I"** produced in step (ii) by contact with an acid in the presence of a suitable solvent or mixture of solvents, optionally isolating the acid addition salt of the compound of formula **I",** and converting the acid addition salt of the compound of formula **I"** into the free base of a compound of formula **I"**.
Surprisingly, the process according to the present invention provides access to the compound of formula **I"** from readily available starting compounds via non-toxic reactions in high yield and high purity. Synthesis in the specified order of steps beneficially involves using compound of formula II", which by itself or by precursor compounds are, via appropriate salt forms, susceptible to easy separation from critical impurities at an early stage of the whole synthesis. Moreover, the desired compound **I"** can be separated from impurities such as residual compound **III"**, as the latter compound does not form salts and therefore the compound **I"** can be isolated directly from organic solvent, if desired. Separation from residual compound **III"** is important as these compounds, for example 4-tert-butyl-4-chlorobutyrophenone, are critical impurities. Step (iii) and particularly the alternative or additional step (iii') is beneficial in view of reliable and efficient purification to yield highly pure free base of a compound of formula **I"**.
The term "leaving group" as used herein means any atom or group of atoms with the proviso that those molecular fragments depart in a heterolytic bond cleavage that is in form of anions or neutral molecules during a substitution or displacement reaction which are typically, but not exclusively, halides or sulfonates as well as water, ammonia, or alcohols.
The term "acid addition salt" as used herein refers to the reaction product of a non-protonated amine base such as compounds of general formulas **I** or **II** respectively with an acid, which may be added to the reaction mixture or emerges during the actual conversion. For example, during the reaction of a compound of formula **II"** with a compound of formula **III"'** one molar equivalent of the corresponding acid HL, wherein L is defined as above, is generated during the substitution reaction.
The terms "acid" and "base" as used herein refer to brønsted acids and brønsted bases that are proton donators and acceptors, respectively.
The term "auxiliary base" as used herein refers to brønsted bases, that is, proton acceptors that differ from compounds of formula **I** and **II** respectively.
The term "solvent" as used herein refers to a polar or non-polar liquid or mixture of liquids that at least partially dissolve a solid, liquid, or gaseous solute, resulting in a solution, emulsion, or a suspension.
(2) The process according to item (1), wherein step (iii) comprises contacting with an acid, from which the corresponding acid addition salt is to be formed, followed by neutralization and optionally recrystallization from a suitable solvent to afford pure free base compound **I'**.
(3) The process according to item (1) or (2), wherein any one of said acid addition salts is independently selected from the group of pharmaceutically acceptable salts.
The term "pharmaceutically acceptable salts" as used herein refers to acid addition salts of the compound of formula **I** or formula **I"** that are obtained by salt formation of said compound of formula **I** or formula **I"** with organic or inorganic acids, preferably, but not exclusively, selected from the group of hydrochloric acid, sulfuric acid, phosphoric acid, glutamic acid, (+)-L-tartaric acid, citric acid, (-)-L-malic acid, DL-lactic acid, L-ascorbic acid, succinic acid, adipic acid, acetic acid, stearic acid, carbonic acid, thiocyanic acid, glycerol-phosphoric acid, L-aspartic acid, maleic acid, fumaric acid, galactaric acid, D-glucuronic acid, glycolic acid, D-glucoheptonic acid, hippuric acid, D-gluconic acid, glutaric acid, sebacic acid, capric (decanoic) acid, lauric acid, palmitic acid, alginic acid, benzoic acid, nicotinic acid, propionic acid, caprylic (octanoic) acid, naphthalene-1,5-disulfonic acid, ethane-1,2-, disulfonic acid, cyclamic acid, p-toluenesulfonic acid, methanesulfonic acid, dodecylsulfuric acid, naphthalene-2-sulfonic acid, benzenesulfonic acid, oxalic acid, 2-hydroxy ethanesulfonic acid, ethanesulfonic acid, pannoic (embonic) acid, 2-oxo glutaric acid, 1-hydroxy-2-naphthoic acid, malonic acid, gentisic acid, lactobionic acid, (-)-L-pyroglutamic acid, oleic acid, (+)-camphoric acid, isobutyric acid, orotic acid.
(4) The process according to any one of the preceding items, wherein any one of said acid addition salts are independently chosen to be respectively soluble in hot solvent solution, preferably above 60°C and more preferably at the solvent's boiling or refluxing temperature, but are subject to precipitation upon decreasing the temperature, preferably at room temperature at about 20-30 °C or below.
(5) The process according to any one of the preceding items, wherein any one of said acid addition salts is independently selected from an oxalate salt, a toluenesulfonate salt and a phosphate salt.
(6) The process according to any one of the preceding items, wherein crude free base of the compound of formula **I"** obtained in step (ii) is provided as a solution in a solvent; and said crude free base of the compound of formula **I'** is directly converted into an acid addition salt thereof by precipitation or crystallization from the solution.
(7) The process according to any one of the preceding items, wherein the precipitation or crystallization of step (iii) is carried out from the mother liquor of the previous reaction step (ii).
(8) The process according to any one of the preceding items, wherein the reactions are performed under an inert atmosphere, preferably under nitrogen atmosphere.
(9) The process according to any one of the preceding items, wherein step (ii) comprises:
**a')** providing respectively a compound of formula **II"** or an acid addition salt thereof, optionally dissolved in a first solvent, preferably adding an auxiliary base and/or a second solvent, and a compound of formula **III"'**,
**b')** heating the mixture obtained in step a'), preferably to reflux temperature, even more preferably under agitation until complete conversion is observed,
**c')** optionally and preferably allowing the mixture to cool, more preferably to ambient temperature, and preferably diluting the mixture with a third solvent,
**d')** adding water, optionally an auxiliary substance selected from sodium chloride and a proton donor, preferably as an aqueous solution, more preferably under agitation and separating the phases,
**e')** optionally repeating the procedure as described in step **d')**, more preferably repeating said procedure twice,
**f')** adding a proton donor to the organic phase obtained in step d') or optional step e'), optionally seeding the mixture in order to induce crystallisation, and allowing an acid addition salt of a compound of formula **I"** to precipitate.
The proton donor optionally used in step d') may be the same as, or different from, the proton donor used in step f'). The proton donor used in step f') is selected from the acid form of the anion intended to precipitate as the acid addition salt.
The term "seeding" as used herein means any conditions known in the art which may assist in the induction of crystallisation, such as adding a seed crystal, scratching a glass surface in contact with the mixture resulting from step f') in order to promote the formation of seed crystals, and/or cooling the mixture resulting from step f') to or below ambient temperature. Preferably, seeding is effected by adding a seed crystal or crystals.
This preferred embodiment of the present invention relates to a powerful purification method for the quantitative separation of unreacted compounds of formula **III"'**. Furthermore, the reaction product is directly obtained from the reaction mixture in high yields and a purity greater than 97 %. The concept of the present invention avoids distillation procedures as well as chromatographic methods. This beneficial embodiment does not only contribute to a high quality product but also significantly facilitates the process compared to previously reported procedures.
(10) The process according to item (9), wherein the first and third solvents and the optional second solvent are selected from the group of polar and non-polar organic solvents or mixtures thereof, preferably from the group of alcohols, ethers, ketones, aromatic hydrocarbons, nitriles, esters, halocarbons and/or formamides.
The first and third solvents and the optional second solvent may be the same or different. The first and optional second solvents used in step a') are preferably selected from the group consisting of MiBK, DMF, toluene, acetone, methyl ethyl ketone, ethyl acetate, acetonitrile, and mixtures thereof, more preferably MiBK, toluene, or mixtures of toluene and DMF, whereas the preferred solvents in step c') are MiBK and toluene.
(11) The process according to any one of the preceding items, wherein the acid addition salt of a compound of formula **II"** is a hydrohalide salt (preferably a hydrochloride or hydrobromide, in particular hydrochloride), oxalate salt, a toluenesulfonate salt and a phosphate salt, preferably the toluenesulfonate salt.
(12) The process according to any one of the preceding items, wherein L in compounds of formula **III"'** is selected from the group consisting of halides such as chloride, bromide or iodide, and sulfonates such as methyl sulfonate, benzene sulfonate, and *para*-toluene sulfonate.
(13) The process according to any one of the preceding items, wherein the auxiliary base is selected from the group of organic and inorganic bases, preferably amines, acetates, alkoxides, hydroxides, hydrgogen carbonates, carbonates, mono- and dihydrogen phosphates, more preferably hydrogen carbonates, even more preferably sodium acetate, triethyl amine, potassium hydrogen carbonate and sodium hydrogen carbonate, and in particular potassium hydrogen carbonate and sodium hydrogen carbonate.
(14) The process according to any one of the preceding items, wherein in step (iii)/(iii') the free base of the compound of formula **I"** is isolated and converted into the acid addition salt of the compound of formula **I"** comprising the steps of:
**g')** concentrating the organic phase obtained in step d') or optionally e'), preferably under reduced pressure, more preferably at reflux temperature, and optionally adding an appropriate solvent and heating the mixture,
**h')** optionally removing insolubles, preferably by filtration, cooling, and allowing the crude free base of a compound of formula **I"** to precipitate,
**i')** isolating, preferably washing, and optionally drying the solid obtained in step h') and
**j')** optionally re-crystallizing the solid obtained in step i') from an appropriate solvent and optionally drying the solid
**k')** dissolving the solid obtained in step i') or j') respectively in an appropriate solvent, adding a proton donor, optionally seeding the mixture in order to induce crystallisation, and allowing an acid addition salt of a compound of formula **I"** to precipitate.
The term "free base" as used herein refers to an electronically neutral primary, secondary, or tertiary amine base such as a compound of formula **I"** in its non-protonated form.
This preferred embodiment of the present invention relates to an optional purification sequence for the separation of related substances, in particular to the separation of compounds of general formula **II"** and **III"'**.
(15) The process according to item (14), wherein the solvent of step g') and j') is selected from the group consisting of polar and non-polar organic solvents or mixtures thereof, whereas the solvents are preferably selected from the group of alcohols, more preferably linear or branched C₁ to C₄-alcohols, such as ethanol, n-propanol or 2-propanol, ketones, such as methyl ethyl ketone, MiBK, and acetone or ethers such as methyl tert-butyl ether and diisopropyl ether, esters such as ethyl acetate, aromatic hydrocarbons such as toluene and alicyclic hydrocarbons such as cylcohexane,
(16) The process according to item (14) and/or (15), wherein the solvent of step k') is selected from the group consisting of the solvents recited in item (15) and nitriles and amides,preferably acetonitrile or DMF .
(17) The process according to items (9) and (14), wherein said proton donor used in item (9)f') and/or item (14)k') is selected from the group of acids that form pharmaceutically acceptable salts as defined in item (3), preferably as defined in items (4) or (5).
(18) The process according to any one of items (9), (14) and (17), wherein said proton donator is added at -15 °C to 80 °C, preferably at 10 °C to 60 °C, and in particular at approx. 25 °C.
(19) The process according to any one of the preceding items, wherein the acid addition salt of a compound of formula **I"** is re-crystallised comprising the steps of:
**l')** providing the acid addition salt of a compound of formula **I"**, and adding an appropriate solvent or mixture of solvents and preferably heating the mixture, more preferably to reflux temperature,
**m')** optionally filtering the mixture, and allowing the solution to cool, optionally seeding the mixture in order to induce crystallisation, and
**n')** allowing said acid addition salt of a compound of formula **I"** to precipitate or crystallize.
(20) The process according to item (19), wherein the solvent is selected from the group of polar organic solvents or mixtures thereof.
(21) The process according to item (20), wherein the solvent is selected from the group consiting of alcohols and nitriles, preferably from linear or branched C₁ to C₄-alcohols and acetonitrile, more preferably from methanol or ethanol.
(22) The process according to any one of the preceding items, wherein the free base or the acid addition salt of the compound of formula **I"** is allowed to precipitate or to crystallize, preferably under agitation and optionally under cooling.
(23) The process according to any one of the preceding items, wherein said free base or acid addition salt of the compound of formula **I"** is isolated, preferably by filtration, and preferably washed, then optionally dried.
(24) The process according to any one of the preceding items, wherein the acid addition salt of the compound of formula **I'** or **I"** is crystalline.
(25) The process according to any one of the preceeding items, wherein the acid addition salt is selected from the phosphate, the toluene sulfonate and the oxalate salt of the compound of formula **I"**.
(26) Use of an acid addition salt of benzhydroxylpiperidine of formula II" 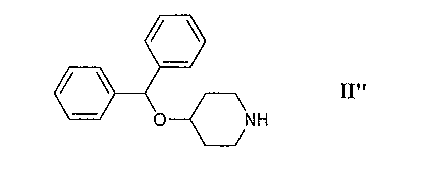 for preparing a crystalline form of the free base compound of formula **I"**, wherein said acid addition salt is selected from an oxalate salt, a toluenesulfonate salt and a phosphate salt, and is preferably the toluenesulfonate salt: 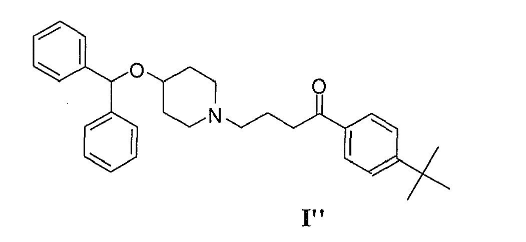 (27) An acid addition salt of benzhydroxylpiperidine of formula II" 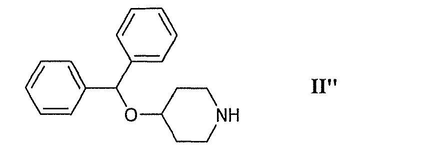 wherein said acid addition salt is selected from an oxalate salt, a toluenesulfonate salt and a phosphate salt, preferably is the toluenesulfonate salt.
(28) A process for preparing a crystalline form of the free base compound of formula **I"**, 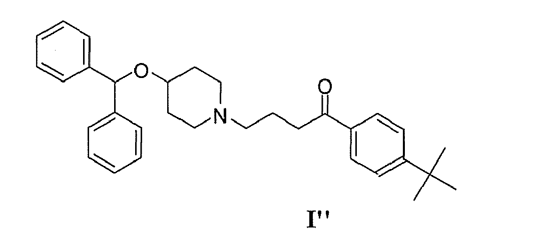 wherein an acid addition salt of the compound of formula **I"** is reacted with a proton acceptor in the presence of at least one suitable solvent, wherein said acid addition salt is selected from an oxalate salt, toluenesulfonate salt or phosphate salt, and then allowing said free base of formula **I"** to precipitate or crystallize.
The term "proton acceptor" has the typical meaning known in the art as a brønsted base, distinct from compounds of formula **I** and **II** respectively.
Surprisingly, the use according to item (26) and the process according to item (28) provides access to the compound of formula **I"** in high yields and high purity. The specified salt forms provide improved embodiments of the present invention, as they are susceptible to easy separation from critical impurities by being soluble at high temperatures while being readily precipitated upon cooling, thereby enabling reliable, efficient and simple purification schemes without a need for laborious purification methods such as chromatography.
(29) The process according to item (28) comprising the steps of :
**o')** providing the acid addition salt of the compound of formula **I"**, adding water and/or an organic solvent or mixtures thereof, preferably with stirring, optionally with heating, and adding the proton acceptor, preferably as an aqueous solution,
**p')** optionally cooling the mixture, preferably to -20 °C to 40 °C, more preferably to 0 °C to 35 °C and particularly to 0 °C to approximately 25 °,
**q')** preferably adjusting the pH to a value between 7 and 7.5, preferably under agitation, optionally seeding the mixture in order to induce crystallisation, and allowing the free base of a compound of formula **I"** to precipitate,
**r')** optionally allowing the solid to age, preferably under agitation, and isolating, preferably washing, and optionally drying the solid;
Or alternative to steps p' to r':
**s')** separating the organic phase obtained in step o'), extracting the aqueous layer, combining the organic phases and evaporating the solvent, and
**t')** isolating and optionally drying the solid obtained in step s').
(30) The process according to item (28) or (29), wherein the solvent is water and/or an organic solvent selected from the group of linear or branched C₁-C₄ alcohols, aromatic hydrocarbons, halocarbons, ethers, ketones, and esters, wherein the organic solvent is more preferably selected from methanol, ethanol, 2-propanol, toluene, dichloromethane, methyl tert-butyl ether, MiBK, acetone orethyl acetate.
(31) The process according to any one of items (28) to (30), wherein the proton acceptor is selected from the group of inorganic bases, such as potassium hydroxide, aqueous ammonia, sodium hydroxide, sodium carbonate or potassium carbonate
(32) The process according to any one of items (28) to (31), wherein said free base is isolated, preferably by filtration, preferably washed, and optionally dried.
(33) The process according to any one of items (28) to (32), further defined by a process as specified in any one of items (1) to (24).
(34) The process according to any one of the preceding items, wherein finally yielded free base of a compound of formula **I"** is characterized by an impurity profile of not more than 0.10 % of each individual impurity and gives a colourless, clear dichloromethane solution.
(35) The process according to any one of the preceding items, wherein the acid addition salt of the compound of formula **I"** has a purity of at least 80 %, preferably not less than 97 %, and even more preferably greater than 99.4 %.
(36) Crystalline free base of formula **I"** exhibiting an X-ray powder diffraction with at least the following significant signals at the indicated d-values, respectively with a tolerance of ± 0.2 (2-Theta°): 16.1, 16.9, 18.8 and 19.4.
(37) Crystalline free base of formula **I"** according item (36) exhibiting an X-ray powder diffraction with at least the following significant signals at the indicated d-values, respectively with a tolerance of ± 0.2 (2-Theta °): 16.1, 16.9, 17.1, 18.8, 19.4, 22.0 and 23.8..
(38) Crystalline free base of formula **I"** according item (36) or (37) exhibiting an X-ray powder diffraction pattern specified in Table 4, respectively with a tolerance of ± 0.2 (2-Theta °):

**Table 4: X-ray powder diffraction data for 1-[4-(1,1-dimethylethyl)phenyl]-4-[4-(diphenylmethoxy)-1-piperidinyl]-1-butanone I".**

| **Angle 2-Theta °** | **d value Angstrom** |
|---|---|
| 5.701 | 15.49072 |
| 9.837 | 8.98392 |
| 11.531 | 7.66776 |
| 12.589 | 7.02562 |
| 13.292 | 6.65555 |
| 14.022 | 6.31096 |
| 14.876 | 5.95033 |
| 16.062 | 5.51361 |
| 16.893 | 5.24436 |
| 17.112 | 5.17769 |
| 17.312 | 5.11811 |
| 17.800 | 4.97886 |
| 18.467 | 4.80075 |
| 18.776 | 4.72237 |
| 19.359 | 4.58135 |
| 19.842 | 4.47104 |
| 21.000 | 4.22695 |
| 21.390 | 4.15079 |
| 21.953 | 4.04547 |
| 22.803 | 3.89662 |
| 23.184 | 3.83354 |
| 23.775 | 3.73953 |
| 24.138 | 3.68410 |
| 24.513 | 3.62851 |
| 24.940 | 3.56741 |
| 25.281 | 3.52007 |
| 25.841 | 3.44501 |
| 26.094 | 3.41218 |
| 26.721 | 3.33354 |
| 27.354 | 3.25776 |
| 27.940 | 3.19079 |
| 28.502 | 3.12909 |
| 29.974 | 2.97872 |
| 30.380 | 2.93984 |
| 30.948 | 2.88714 |
| 31.758 | 2.81537 |
| 33.275 | 2.69040 |
| 34.534 | 2.59516 |
| 36.012 | 2.49193 |
| 36.616 | 2.45221 |
| 38.314 | 2.34733 |
| 39.443 | 2.2827 |

(39) The crystalline free base of formula **I"** according to any one of items (36) to (38), obtainable by a process according to any of items (1) to (34).
(40) A process to prepare a pure pharmaceutically acceptable salt of a compound of formula **I"**, comprising the steps of
carrying out a process according to any one of items (1) to (35) to prepare pure free base of the compound of formula **I"**; and
reacting the free base with an organic or inorganic acid of a pharmaceutically acceptable anion to give the corresponding pharmaceutically acceptable salt.
(41) A process to prepare a pharmaceutical composition comprising a compound of formula **I"** or a pharmaceutically acceptable salt thereof, comprising the steps of carrying out a process according to any one of items (1) to (35) or a process according to item (40) to prepare the free base of a compound of formula **I"** or a pharmaceutically acceptable salt thereof; and
mixing said free base of the compound of formula **I"** or a pharmaceutically acceptable salt thereof with a pharmaceutically acceptable excipient.
(42) The process according to item (41), wherein a free base of the compound of formula **I"** according to any one of items (36) to (38) is used.
(43) A crystalline phosphate salt of a compound of formula **I"**
(44) The phosphate salt according to item (43), exhibiting an X-ray powder diffraction with at least the following significant signals at the indicated d-values, respectively with a tolerance of ± 0.2 (2-Theta °): 7.2, 14.0, 14.5, 19.0 and 20.1.
(45) The phosphate salt according to item (44), exhibiting a X-ray powder diffraction pattern specified in Table 1, respectively with a tolerance of ± 0.2 (2-Theta:

**Table 1: X-ray powder diffraction data for 1-[4-(1,1-dimethylethyl)phenyl]-4-[4-(diphenylmethoxy)-1-piperidinyl]-1-butanone Phosphate I" · H₃PO₄**

| **Angle 2-Theta°** | **d value Angstrom** |
|---|---|
| 5.334 | 16.55567 |
| 6.408 | 13.78218 |
| 7.193 | 12.28008 |
| 8.248 | 10.71084 |
| 9.683 | 9.12652 |
| 12.082 | 7.31948 |
| 12.907 | 6.85337 |
| 13.959 | 6.33931 |
| 14.546 | 6.08467 |
| 16.355 | 5.41537 |
| 16.745 | 5.29028 |
| 18.439 | 4.80778 |
| 18.987 | 4.67030 |
| 19.492 | 4.55039 |
| 20.098 | 4.41449 |
| 20.864 | 4.25420 |
| 21.725 | 4.08746 |
| 22.896 | 3.88109 |
| 23.506 | 3.78164 |
| 24.270 | 3.66440 |
| 25.316 | 3.51526 |
| 25.720 | 3.46094 |
| 26.997 | 3.30010 |
| 27.473 | 3.24394 |
| 27.959 | 3.18868 |
| 28.907 | 3.08626 |
| 26.997 | 3.30010 |
| 27.473 | 3.24394 |
| 27.959 | 3.18868 |
| 28.907 | 3.08626 |
| 29.656 | 3.00996 |
| 30.590 | 2.92016 |
| 31.629 | 2.82654 |
| 33.541 | 2.66962 |
| 35.740 | 2.51028 |
| 36.881 | 2.43519 |
| 38.241 | 2.35168 |

(46) A crystalline oxalate salt of a compound of formula **I"**.
(47) The oxalate salt according to item (46), exhibiting a X-ray powder diffraction with at least the following significant signals at the indicated d-values, respectively with a tolerance of ± 0.2 (2-Theta °): 16.1, 17.0, 18.8, 19.4 and 31.6.
(48) The oxalate salt according to item (46), exhibiting a X-ray powder diffraction pattern specified in Table 1, respectively with a tolerance of ± 0.2 (2-Theta °):

**Table 2: X-ray powder diffraction data for 1-[4-(1,1-dimethylethyl)phenyl]-4-[4-(diphenylmethoxy)-1-piperidinyl]-1-butanone oxalate I"·H₂C₂O₄**

| **Angle 2-Theta°** | **d value Angstrom** |
|---|---|
| 5.749 | 15.36112 |
| 9.862 | 8.96160 |
| 11.540 | 7.66167 |
| 12.569 | 7.03711 |
| 13.280 | 6.66185 |
| 14.038 | 6.30367 |
| 14.890 | 5.94488 |
| 16.075 | 5.50908 |
| 16.931 | 5.23253 |
| 17.189 | 5.15446 |
| 17.796 | 4.97996 |
| 18.404 | 4.81691 |
| 18.793 | 4.71798 |
| 19.351 | 4.58317 |
| 19.860 | 4.46693 |
| 20.222 | 4.38767 |
| 21.000 | 4.22695 |
| 21.415 | 4.14590 |
| 21.944 | 4.04722 |
| 22.836 | 3.89108 |
| 23.760 | 3.74175 |
| 24.140 | 3.68377 |
| 24.477 | 3.63376 |
| 24.993 | 3.55999 |
| 25.341 | 3.51179 |
| 25.882 | 3.43964 |
| 26.764 | 3.32825 |
| 27.371 | 3.25580 |
| 28.510 | 3.12830 |
| 30.000 | 2.97619 |
| 30.748 | 2.90545 |
| 31.559 | 2.83264 |
| 32.280 | 2.77100 |
| 33.100 | 2.70424 |
| 33.482 | 2.67426 |
| 34.363 | 2.60769 |
| 35.269 | 2.54270 |
| 36.039 | 2.49016 |
| 36.664 | 2.44911 |
| 38.468 | 2.33831 |
| 39.446 | 2.28254 |

(49) A crystalline toluene sulfonate salt of a compound of formula **I"**.
(50) The toluene sulfonate salt according to item (49), exhibiting an X-ray powder diffraction with at least the following significant signals at the indicated d-values, respectively with a tolerance of ± 0.2 (2-Theta °): 4.3, 19.4, 20.1 and 23.7.
(51) The toluene sulfonate salt according to item (49), exhibiting an X-ray powder diffraction pattern specified in Table 1, respectively with a tolerance of ± 0.2 (2-Theta °):

**Table 3: X-ray powder diffraction data for 1-[4-(1,1-dimethylethyl)phenyl]-4-[4-(diphenylmethoxy)-1-piperidinyl]-1-butanone tosylate I"· HOSO₃C₆H₄CH₃-**

| **Angle 2-Theta °** | **d value Angstrom** |
|---|---|
| 4.345 | 20.32116 |
| 8.793 | 10.04902 |
| 9.198 | 9.60700 |
| 10.623 | 8.32162 |
| 11.241 | 7.86495 |
| 11.735 | 7.53503 |
| 11.993 | 7.37345 |
| 13.230 | 6.68660 |
| 13.891 | 6.37015 |
| 14.281 | 6.19686 |
| 15.591 | 5.67922 |
| 16.283 | 5.43922 |
| 16.850 | 5.25746 |
| 17.498 | 5.06435 |
| 18.472 | 4.79944 |
| 19.363 | 4.58040 |
| 20.116 | 4.41056 |
| 21.406 | 4.14766 |
| 21.681 | 4.09568 |
| 22.107 | 4.01767 |
| 23.650 | 3.75901 |
| 24.100 | 3.68977 |
| 25.058 | 3.55083 |
| 25.443 | 3.49801 |
| 25.937 | 3.43249 |
| 27.207 | 3.27503 |
| 27.597 | 3.22962 |
| 27.988 | 3.18545 |
| 28.848 | 3.09240 |
| 29.381 | 3.03751 |
| 30.212 | 2.95578 |
| 31.282 | 2.85714 |
| 32.439 | 2.75781 |
| 33.373 | 2.68273 |
| 34.356 | 2.60818 |
| 35.485 | 2.52773 |
| 36.998 | 2.42776 |
| 37.785 | 2.37900 |

According to the beneficial embodiments of items (43) to (51), highly pure intermediates are provided which are particularly useful as pharmaceutically acceptable ingredients themselves, or for the preparation of a highly pure compound of formula **I"**, or for a final conversion to other pharmaceutically acceptable salts. The selected crystalline salts respectively allow highly efficient purification concepts by simple precipitation or crystallization from appropriate solvents, thereby providing access to the respective salts in highly pure and isolated form. In particular in the conversion into the salt of the compound of formula **I"** in accordance with a preferred embodiment, substantial lowering of solubility and thus yield of precipitation in an appropriate solvent can be enhanced such as in C1-C4 alcohol, ketones and esters, wherein the solvent is more preferably selected from methanol, ethanol, 2-propanol, acetone and ethyl acetate and in particular ethanol. Further enhanced purification is possible in the order of the toluenesulfonate salt, the oxalate salt and the phosphate salt.
The yield obtained for salt synthesis typically reflects the solubility of the salts, and thus for example HCl gave no precipitate and is probably the most soluble, whereas toluenesulfonic acid gave a fairly good yield (61.21%) from ethanol and phosphoric acid gave a good yield (98.21 %) from ethanol. The oxalate salt gave a high yield from acetone (89.59%) and is insoluble in most solvents. The following solubility ranking is, therefore, possible. HCl salt > tosylate > phosphate, oxalate.

| Acid | Yield of salt | Solvent |
|---|---|---|
| HCl | 0% | Ethanol |
| Toluenesulfonic acid | 61.21% | Ethanol |
| Phosphoric acid | 98.21% | Ethanol |
| Oxalic acid | 89.59% | Acetone |

Given their usefulness due to their accessible high purity, the crystalline acid addition salts of of items (43) to (51) of the compound of formula **I"** may also be used directly as an active pharmaceutical ingredient in a pharmaceutical formulation.
(52) Use of an acid addition salt of a compound of formula **I"** selected from the group consisting of phosphate, toluenesulfonate and oxalate salts as defined in any one of items (43) to (51) in a process for the manufacture of a pharmaceutically active ingredient.
(53) The use according to item (52), wherein said acid addition salt of a compound of formula **I"** is converted to the free base of the compound of formula **I"** that is used in the manufacture of a pharmaceutical formulation.

### Detailed description of the invention

Beneficial and preferred embodiments of the present invention involve a particular order of synthetic steps and/or the intermediate preparation of acid addition salts of a compound of formula **II"** and/or a compound of formula **I"** as particularly suitable intermediates, to thereby allow unexpectedly efficient, albeit simple, purification schemes. If desired, a final liberation of the corresponding free base and an optional recrystallization from a suitable solvent can afford compound **I"** of pharmaceutical purity. In particular embodiments, new salts of compound **I"** are provided which already exhibit pharmaceutical purity. Other preferred embodiments involve a well-aimed and highly efficient transformation chain "crude free base" → "acid addition salt" → "pure free base". Desirable and feasible pharmaceutical purity means to provide a white or almost-white crystalline form, typically a powder form, at a beneficial purity of at least 99.0 %, wherein related unwanted substances are at a content of 0.1 % or lower. The procedural concept of this embodiment is to achieve the purification of the active pharmaceutical ingredient 1-[4-(1,1-dimethylethyl)phenyl]-4-[4-(diphenylmethoxy)-1-piperidinyl]-1-butanones of formula **I"** to the desired pharmaceutical purity.

Purity values indicated in the present specification means HPLC area %. The term "about" means ± 10% of the respectively indicated value.

An illustrative scheme of a relevant step in the synthesis of the compound of formula **I"** is shown in Scheme 7 below, which e.g. compared to another reaction sequence as depicted from W02009157006 can provide overall better results in terms of both final yield and purity. 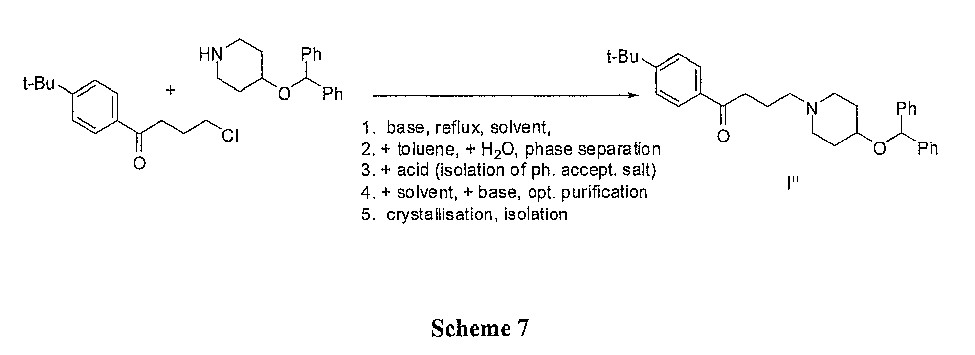 This is manifested in the examples described below, for example when processed via ebastine phosphate (moist) having HPLC purity 99.5 % (Example 9), combined with conversion into pure free ebastine with a final yield of 70 % (calcd. on **II"** obtained from Example. 9) and a HPLC purity of 99.9 % (Example 11); or when processed via oxalate to finally yield 56 % of pure free base **I"** having a HPLC purity of 99.5 % (Example 20); or when processed via tosylate to finally yield 69 % of pure free base **I"** having a HPLC purity of 99.99 % (Example 2).

Salts and especially the tosylate salt of the compound of formula **II"** display particular advantages in the synthesis. Toluenesulfonic acid is the most efficient acid for synthesis of an ether from 4-hydroxypiperidine and benzhydrol. Notably the tosylate is readily formed in solution at elevated temperatures such as at the boiling point or reflux temperature and, surprisingly on cooling to room temperature the tosylate salt precipitates from solution. Alternative purification of compound **II'** as the free base would be much more complicated as large amounts of benzhydrol dimer are typically formed during the etherification, and for direct use of the free base, the toluenesulfonic acid would have to be removed also. Unless the salt and particularly the tosylate salt of the compound of formula **II"** are used for the reaction, the aforementioned impurities would have to be separated from compound **II"** by a time consuming sequence of sodium hydroxide extraction to remove the respective acid such as toluenesulfonic acid, acidic extraction to separate the piperidine derivative from the dimer and neutralization to provide the base. Beneficially, the salt such as the tosylate salt of a compound of formula **II"**, in turn, can be directly used in the synthesis of Ebastine of formula **I"** by use of an excess of potassium carbonate as base in the next step. The desired product is obtained in over 99% purity by HPLC.

An illustrative scheme of a purification concept of the compound of formula **I"** according to a preferred embodiment of the present invention is illustrated in scheme 8 below, involving the preparation of an acid addition salt I"·HB of a corresponding conjugate base B⁻, followed by neutralization with a suitable basic substance 1) and optional recrystallization 2) from a suitable solvent to finally afford compound **I"** of pharmaceutical purity.

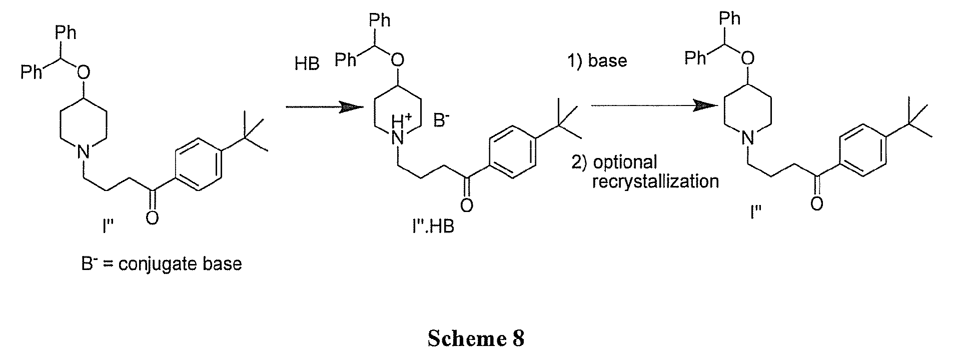

According to preferred embodiments of the present invention, there are thus provided crystalline acid addition salts of a compound of formula **II"** and/or of a compound of formula **I"** as important synthetic intermediates as well as their use in an improved process for the synthesis of pure 1-[4-(1,1-dimethylethyl)phenyl]-4-[4-(diphenylmethoxy)-1-piperidinyl]-1-butanone of formula **I"**.

According to a particular embodiment, highly pure Ebastine of formula **I"** is prepared via an intermediate acid addition salt by reaction of 4-benzhydryloxypiperidine **II"**, or preferably an addition salt thereof such as 4-benzyhydryloxypiperidine hydrochloride **II"·HCl** or the toluenesulfonate salt, and 4'-tert-butyl-4-chloro-butyrophenone **III"** in the presence of a suitable base such as sodium hydrogen carbonate and in a suitable solvent such as methyl isobutyl ketone or preferably in a mixture of solvents, in particular a mixture of DMF and toluene, preferably under nitrogen gas at a temperature in the range of from 5 °C to 150 °C, preferably from 80 °C to 130 °C, for example at about 121 °C for a suitable time period of e.g. 4 to 8 h (scheme 9). Surprisingly, performing the reaction under a nitrogen atmosphere leads to a reduced reaction time as well as suppressing presumed oxidation of 1-[4-(1,1-dimethylethyl)phenyl] -4- [4-(diphenylmethoxy)-1-piperidinyl]-1-butanone or 4-benzhydryloxypiperidine, which can lead to colouration of the product.

The reaction mixture containing ― and thereby efficiently providing — crude compound **I"** is preferably vigorously washed with water or another aqueous solution to remove inorganic material, yielding a solution of compound **I"**. Sodium chloride solution may be used for aqueous extraction to provide improved density discrimination between the organic and aqueous phases. When appropriate an acetic acid wash may be employed to remove traces of the amine **II"**. The organic solution containing the free base **I"** may either (i) be treated directly with proton donors which are preferably selected from the group of acids that form pharmaceutically acceptable salts, such as oxalic acid, oxalic acid dihydrate, phosphoric or toluene sulfonic acid, or (ii) subjected to a solvent exchange to water miscible solvents, such as amides, nitriles, alcohols or ketones, preferably by means of azeotropic removal of the reaction solvents. Alternatively crude base **I"** can be isolated and recrystallized prior to formation of the relevant salt.

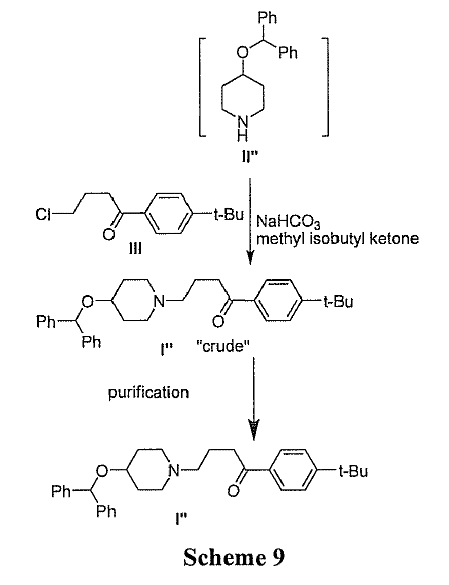

As a key step of "purification" indicated in scheme 9, crude base **I"**, optionally after being isolated and recrystallized, is converted into pure base **I"** via its acid addition salt, wherein said acid addition salt is chosen to be soluble in hot solvent solution, preferably above 60°C and more preferably at the solvent's boiling or refluxing temperature, but is subject to precipitation or crystallization upon decreasing the temperature, preferably at room temperature at about 20-25 °C or below.. The isolation of acid addition salts of **I"** presents distinct advantages over conventional purification methods for the free base such as chromatography as the procedure is scalable, and time- and energy-consuming distillation of the solvent (necessary in solvent exchange or aqueous extraction approaches) is avoided owing to the finding that the acid addition salt precipitates as a substantially pure substance.

The processes according to the present invention thus combines advantages of applicability on a technical scale, simplicity and low cost.

Moreover, the 1-[4-(1,1-dimethylethyl)phenyl]-4-[4-(diphenylmethoxy)-1-piperidinyl]-1-butanone **I"** can be separated from impurities such as residual 4-tert-butyl-4-chlorobutyrophenone **III"** as these compounds do not form salts and the desired compound can be isolated from the organic solvent directly, because the salts used, in particular when oxalate, toluenesulfonate or phosphate is used, are insoluble in appropriate organic solvents. For example when the purification scheme is carried out via the toluenesulfonate salt, it is possible to finally prepare I" of pharmaceutical quality.

The procedures listed above have further advantages as a mildly reactive alkyl chloride is used in the final step of the synthesis, as opposed to the highly reactive, toxic alkylating agents benzhydryl bromide and chloride used in other processes. Additionally, the reaction conditions are sufficiently vigorous that use of a more reactive halide such as an iodide is not necessary and the Finkelstein procedure used in EP0134124A1 becomes superfluous.

Recrystallization can be employed either in tandem or as an alternative means of purification for base **I"**. The crude base can be isolated by distillation of the crude reaction mixture to afford a residue, which is then dissolved in an appropriate solvent such as ethanol or isopropanol preferably with heating, and filtration then gives a super-saturated solution of **I"**, which is preferably seeded with pure **I"** to provide the crystalline free base on cooling. Purity in excess of 99.9% (HPLC relative peak area) could be obtained when ethanol was used as the solvent for recrystallization.

Free base **I"** obtained as disclosed herein was a white, crystalline powder, which was analysed by XRPD. The following X-ray reflection data were obtained. Intensity values are indicated for illustrative purposes only.

**Table 5: X-ray powder diffraction data for 1-[4-(1,1-dimethylethyl)phenyl]-4-[4-(diphenylmethoxy)-1-piperidinyl]-1-butanone**

| **Angle 2-Theta°** | **d value Angstrom** | **Intensity** % |
|---|---|---|
| 5.701 | 15.49072 | 7.8 |
| 9.837 | 8.98392 | 6.3 |
| 11.531 | 7.66776 | 10.5 |
| 12.589 | 7.02562 | 3.1 |
| 13.292 | 6.65555 | 4.7 |
| 14.022 | 6.31096 | 17.7 |
| 14.876 | 5.95033 | 9.9 |
| 16.062 | 5.51361 | 35.2 |
| 16.893 | 5.24436 | 34.2 |
| 17.112 | 5.17769 | 20.1 |
| 17.312 | 5.11811 | 7.9 |
| 17.800 | 4.97886 | 4.9 |
| 18.467 | 4.80075 | 22.9 |
| 18.776 | 4.72237 | 96.4 |
| 19.359 | 4.58135 | 100.0 |
| 19.842 | 4.47104 | 7.4 |
| 21.000 | 4.22695 | 4.4 |
| 21.390 | 4.15079 | 13.6 |
| 21.953 | 4.04547 | 25.9 |
| 22.803 | 3.89662 | 12.1 |
| 23.184 | 3.83354 | 2.5 |
| 23.775 | 3.73953 | 24.2 |
| 24.138 | 3.68410 | 17.5 |
| 24.513 | 3.62851 | 3.0 |
| 24.940 | 3.56741 | 6.7 |
| 25.281 | 3.52007 | 15.3 |
| 25.841 | 3.44501 | 15.0 |
| 26.094 | 3.41218 | 5.5 |
| 26.721 | 3.33354 | 11.8 |
| 27.354 | 3.25776 | 6.6 |
| 27.940 | 3.19079 | 3.2 |
| 28.502 | 3.12909 | 12.1 |
| 29.974 | 2.97872 | 5.9 |
| 30.380 | 2.93984 | 2.2 |
| 30.948 | 2.88714 | 4.3 |
| 31.758 | 2.81537 | 2.0 |
| 33.275 | 2.69040 | 4.5 |
| 34.534 | 2.59516 | 3.2 |
| 36.012 | 2.49193 | 2.4 |
| 36.616 | 2.45221 | 2.7 |
| 38.314 | 2.34733 | 3.0 |
| 39.443 | 2.2827 | 6 |

Salt formation and neutralization however provides a more versatile approach to purification.

Salts have been prepared from isolated **I"** or from **I"** in solution, as described above. Salt recrystallization could be employed to remove occluded material and to separate the amine salt from other salts, such as salts of **II"**.

For example, synthesis of the phosphate salt **I"·H₃PO₄** was accomplished either by treatment of an acetone or a denatured ethanol solution of **I"** with phosphoric acid or by treatment of a solution of crude **I"** with phosphoric acid, followed by seeding the reaction mixture with a crystal of **I"·H₃PO₄**. The resulting salt had the following XRPD characteristics. Intensity values are indicated for illustrative purposes only.

**Table 6: X-ray powder diffraction data for 1-[4-(1,1-dimethylethyl)phenyl]-4-[4-(diphenylmethoxy)-1-piperidinyl]-1-butanone Phosphate I" · H₃PO₄**

| **Angle 2-Theta °** | **d value Angstrom** | **Intensity** % |
|---|---|---|
| 5.334 | 16.55567 | 11.1 |
| 6.408 | 13.78218 | 4.2 |
| 7.193 | 12.28008 | 89.8 |
| 8.248 | 10.71084 | 1.5 |
| 9.683 | 9.12652 | 25.0 |
| 12.082 | 7.31948 | 4.0 |
| 12.907 | 6.85337 | 6.3 |
| 13.959 | 6.33931 | 53.4 |
| 14.546 | 6.08467 | 79.1 |
| 16.355 | 5.41537 | 25.3 |
| 16.745 | 5.29028 | 41.2 |
| 18.439 | 4.80778 | 22.4 |
| 18.987 | 4.67030 | 100 |
| 19.492 | 4.55039 | 13.7 |
| 20.098 | 4.41449 | 46.2 |
| 20.864 | 4.25420 | 26.5 |
| 21.725 | 4.08746 | 28.8 |
| 22.896 | 3.88109 | 9.1 |
| 23.506 | 3.78164 | 17.0 |
| 24.270 | 3.66440 | 17.7 |
| 25.316 | 3.51526 | 16.5 |
| 25.720 | 3.46094 | 13.1 |
| 26.997 | 3.30010 | 16.6 |
| 27.473 | 3.24394 | 4.7 |
| 27.959 | 3.18868 | 3.2 |
| 28.907 | 3.08626 | 7.8 |
| 26.997 | 3.30010 | 17.0 |
| 27.473 | 3.24394 | 4.7 |
| 27.959 | 3.18868 | 3.2 |
| 28.907 | 3.08626 | 7.8 |
| 29.656 | 3.00996 | 2.8 |
| 30.590 | 2.92016 | 6.9 |
| 31.629 | 2.82654 | 4.5 |
| 33.541 | 2.66962 | 6.3 |
| 35.740 | 2.51028 | 5.6 |
| 36.881 | 2.43519 | 4.9 |
| 38.241 | 2.35168 | 11.2 |

The oxalate salt of **I"**, **I"·H₂C₂O₄** was synthesized from **I"** and oxalic acid or oxalic acid dihydrate. The solvents employed were toluene, acetone, ethanol and acetonitrile and isolated yields of the pure salt of over 95% were obtained and a HPLC purity of over 99.5% was observed in some cases.

The salt was characterized as a 1:1 salt by titration and possessed the following XRPD characteristics. Intensity values are indicated for illustrative purposes only.

**Table 7: X-ray powder diffraction data for 1-[4-(1,1-dimethylethyl)phenyl]-4-[4-(diphenylmethoxy)-1-piperidinyl]-1-butanone oxalate I"· H₂C₂O₄**

| **Angle 2-Theta °** | **d value Angstrom** | **Intensity** % |
|---|---|---|
| 5.749 | 15.36112 | 24.7 |
| 9.862 | 8.96160 | 6.6 |
| 11.540 | 7.66167 | 25.5 |
| 12.569 | 7.03711 | 3.2 |
| 13.280 | 6.66185 | 3.8 |
| 14.038 | 6.30367 | 18.6 |
| 14.890 | 5.94488 | 19.5 |
| 16.075 | 5.50908 | 38.7 |
| 16.931 | 5.23253 | 39.7 |
| 17.189 | 5.15446 | 19.2 |
| 17.796 | 4.97996 | 9.4 |
| 18.404 | 4.81691 | 16.3 |
| 18.793 | 4.71798 | 77.1 |
| 19.351 | 4.58317 | 100.0 |
| 19.860 | 4.46693 | 6.7 |
| 20.222 | 4.38767 | 5.8 |
| 21.000 | 4.22695 | 4.9 |
| 21.415 | 4.14590 | 9.4 |
| 21.944 | 4.04722 | 25.9 |
| 22.836 | 3.89108 | 12.0 |
| 23.760 | 3.74175 | 29.0 |
| 24.140 | 3.68377 | 13.3 |
| 24.477 | 3.63376 | 3.5 |
| 24.993 | 3.55999 | 5.4 |
| 25.341 | 3.51179 | 20.9 |
| 25.882 | 3.43964 | 22.3 |
| 26.764 | 3.32825 | 12.8 |
| 27.371 | 3.25580 | 4.2 |
| 28.510 | 3.12830 | 13.8 |
| 30.000 | 2.97619 | 15.7 |
| 30.748 | 2.90545 | 20.3 |
| 31.559 | 2.83264 | 74.9 |
| 32.280 | 2.77100 | 6.1 |
| 33.100 | 2.70424 | 6.0 |
| 33.482 | 2.67426 | 6.5 |
| 34.363 | 2.60769 | 28.0 |
| 35.269 | 2.54270 | 2.0 |
| 36.039 | 2.49016 | 7.8 |
| 36.664 | 2.44911 | 9.2 |
| 38.468 | 2.33831 | 22.9 |
| 39.446 | 2.28254 | 8.5 |

Recrystallization of **I"**·**HOSO₃Tol** from acetonitrile or **I"**·**H₂C₂O₄** from methanol resulted in improved HPLC purity. The XRPD reflection data of the tosylate salt are given in Table 8 below. Intensity values are indicated for illustrative purposes only.

**Table 8: X-ray powder diffraction data for 1-[4-(1,1-dimethylethyl)phenyl]-4-[4-(diphenylmethoxy)-1-piperidinyl]-1-butanone tosylate I"·HOSO₃C₆H₄CH₃.**

| **Angle 2-Theta °** | **d value Angstrom** | **Intensity** % |
|---|---|---|
| 4.345 | 20.32116 | 100.0 |
| 8.793 | 10.04902 | 1.5 |
| 9.198 | 9.60700 | 5.6 |
| 10.623 | 8.32162 | 5.3 |
| 11.241 | 7.86495 | 5.2 |
| 11.735 | 7.53503 | 4.8 |
| 11.993 | 7.37345 | 3.3 |
| 13.230 | 6.68660 | 12.1 |
| 13.891 | 6.37015 | 3.8 |
| 14.281 | 6.19686 | 18.1 |
| 15.591 | 5.67922 | 5.4 |
| 16.283 | 5.43922 | 26.3 |
| 16.850 | 5.25746 | 11.6 |
| 17.498 | 5.06435 | 16.8 |
| 18.472 | 4.79944 | 16.8 |
| 19.363 | 4.58040 | 30.5 |
| 20.116 | 4.41056 | 41.0 |
| 21.406 | 4.14766 | 14.6 |
| 21.681 | 4.09568 | 15.9 |
| 22.107 | 4.01767 | 10.4 |
| 23.650 | 3.75901 | 35.3 |
| 24.100 | 3.68977 | 20.0 |
| 25.058 | 3.55083 | 9.5 |
| 25.443 | 3.49801 | 6.5 |
| 25.937 | 3.43249 | 10.0 |
| 27.207 | 3.27503 | 14.5 |
| 27.597 | 3.22962 | 8.8 |
| 27.988 | 3.18545 | 6.3 |
| 28.848 | 3.09240 | 5.4 |
| 29.381 | 3.03751 | 2.0 |
| 30.212 | 2.95578 | 3.0 |
| 31.282 | 2.85714 | 2.0 |
| 32.439 | 2.75781 | 4.3 |
| 33.373 | 2.68273 | 2.3 |
| 34.356 | 2.60818 | 2.1 |
| 35.485 | 2.52773 | 3.3 |
| 36.998 | 2.42776 | 5.1 |
| 37.785 | 2.37900 | 3.2 |

Various neutralization processes were developed for **I"** salts, providing a range of purification techniques for **I"** free base. In one of the simplest neutralization procedures, the salt such as the oxalate was suspended in water and an auxiliary base such as ammonia was added with stirring and the free base gradually precipitated from the well-stirred heterogeneous mixture. Interestingly, 1-[4-(1,1-dimethylethyl)phenyl]-4-[4-(diphenylmethoxy)-1-piperidinyl]-1-butanone obtained in this manner was microcrystalline. For example 52.36% of the sample had a particle size under 400 µm, and crystals were hard to distinguish by microscopy. The XRPD reflection data are given in Table 9 below. Intensity values are indicated for illustrative purposes only. Microcrystalline 1-[4-(1,1-dimethylethyl)phenyl]-4-[4-(diphenylmethoxy)-1-piperidinyl]-1-butanone could be advantageous for drug delivery purposes as solubility differences between different crystal forms and crystal sizes of drugs are often seen (Polymorphism in the Pharmaceutical Industry, Hilfiker Ed., Wiley-VCH, Weinheim, 2006).

**Table 9: X-ray powder diffraction data for 1-[4-(1,1-dimethylethyl)phenyl]-4-[4-(diphenylmethoxy)-1-piperidinyl]-1-butanone I"**

| **Angle 2-Theta°** | **d value Angstrom** | **Intensity** % |
|---|---|---|
| 2,824 | 31,25793 | 3,8 |
| 5,634 | 15,67325 | 8,0 |
| 8,453 | 10,45162 | 7,9 |
| 9,873 | 8,95191 | 5,7 |
| 10,871 | 8,13201 | 6,7 |
| 11,538 | 7,66326 | 7,7 |
| 11,770 | 7,51257 | 4,1 |
| 12,573 | 7,03479 | 2,2 |
| 13,302 | 6,65074 | 5,6 |
| 14,041 | 6,30217 | 13,2 |
| 14,909 | 5,93719 | 5,9 |
| 15,498 | 5,71305 | 3,5 |
| 16,155 | 5,48214 | 28,6 |
| 17,066 | 5,19153 | 35,1 |
| 17,869 | 4,95997 | 3,5 |
| 18,446 | 4,80604 | 20,6 |
| 18,880 | 4,69664 | 73,3 |
| 19,073 | 4,64934 | 65,0 |
| 19,379 | 4,57678 | 100,0 |
| 19,877 | 4,46326 | 18,2 |
| 20,303 | 4,37042 | 3,0 |
| 20,841 | 4,25875 | 13,2 |
| 21,594 | 4,11209 | 66,7 |
| 22,020 | 4,03340 | 40,5 |
| 22,311 | 3,98147 | 11,9 |
| 22,805 | 3,89623 | 29,7 |
| 23,365 | 3,80418 | 13,1 |
| 23,780 | 3,73871 | 17,7 |
| 24,160 | 3,68076 | 9,7 |
| 24,645 | 3,60943 | 5,5 |
| 25,025 | 3,55548 | 14,6 |
| 25,322 | 3,51436 | 15,3 |
| 25,725 | 3,46025 | 31,2 |
| 26,075 | 3,41457 | 9,1 |
| 26,854 | 3,31734 | 13,3 |
| 28,508 | 3,12850 | 7,4 |
| 29,539 | 3,02163 | 1,7 |
| 30,081 | 2,96843 | 4,0 |
| 30,467 | 2,93162 | 3,5 |
| 31,374 | 2,84895 | 16,0 |
| 32,102 | 2,78592 | 6,5 |
| 32,751 | 2,73222 | 5,3 |
| 33,258 | 2,69169 | 6,4 |
| 34,497 | 2,59785 | 4,6 |
| 35,110 | 2,55386 | 5,1 |
| 35,580 | 2,52120 | 2,9 |
| 36,237 | 2,47695 | 9,6 |
| 37,919 | 2,37091 | 4,0 |
| 38,380 | 2,34346 | 4,3 |

Distinct from a low solubility at relatively low temperature conditions such as room temperature or below, the unexpected solubility of the acid addition salts in appropriate solvents under certain conditions, for example 1-[4-(1,1-dimethylethyl)phenyl]-4-[4-(diphenylmethoxy)-1-piperidinyl]-1-butanone phosphate **I"**·H₃PO₄ in ethanol at reflux temperature, allows for the purification of the salt as a part of the neutralization process. Thus, an ethanol mixture of 1-[4-(1,1-dimethylethyl)phenyl]-4-[4-(diphenylmethoxy)-1-piperidinyl]-1-butanone phosphate **I"**·H₃PO₄ and aqueous sodium hydroxide is heated to reflux and then cooled to provide 1-[4-(1,1-dimethylethyl)phenyl]-4-[4-(diphenylmethoxy)-1-piperidinyl]-1-butanone **I"** after precipitation (Example 6). The overall yield for this process from 4-benzhydryloxypiperidine **II"** is 69.65 %.

Alternative neutralization conditions can be employed. The neutralization reactions are usually performed with auxiliary bases such as sodium or potassium hydroxide, or with potassium carbonate and provide access to 1-[4-(1,1-dimethylethyl)phenyl]-4-[4-(diphenylmethoxy)-1-piperidinyl]-1-butanone **I"**, which can then be extracted into a variety of organic solvents such as toluene, dichloromethane, ethyl acetate or methyl tert-butyl ether.

Neutralization can advantageously be performed either such that neither the organic starting material nor the organic product are dissolved in the solvent, or followed by heating so that the resulting 1-[4-(1,1-dimethylethyl)phenyl]-4-[4-(diphenylmethoxy)-1-piperidinyl]-1-butanone **I"** can be dissolved in a solvent such as ethanol, allowing for subsequent crystallization on cooling if desired. By this process 1-[4-(1,1-dimethylethyl)phenyl]-4-[4-(diphenylmethoxy)-1-piperidinyl]-1-butanone oxalate **I"**·H₂C₂O₄ neutralization and purification has been conducted in ethanol.

Similar neutralization protocols are possible with other salts such as the tosylate salt.

### Experimental Part

### Methods

**Heavy metals.** According to USP (231, method II), **Sulphated ash.** According to Ph Eur. (2.4.14). **IR.** FTIR-spectrometer Spectrum one (Perkin-Elmer); according to Ph. Eur (2.2.24,KBr). **DSC.** DSC822 (Mettler), inert gas; nitrogen, temperature program from 30 °C to 400 °C/heating rate: 10 °C/min. **Water content.** Titrando 835 (Metrohm) with double-Pt-electrode; Karl-Fischer.titration. **MP,** B545 (Buchi). **Residual solvents.** Gas chromatograph 6890 (Agilent) with head space sampler (Agilent); head space gas chromatography, column DB-624: 30 m length, 0.32 mm ID, 1.8 um film thickness, temperature program from 40 °C to 250 °C. **NMR**. Bruker AVANCE 300 spectrometer at 301 K. **XRPD.** Transmission diffractometer STADI P (STOE & Cie., Darmstadt, Germany) using Ge-monochromated CuK_{α1} radiation (1.5406 A) in Debye-Scherrer.geometry. **Gas Chromatography.** The analysis was carried out by using a GC-system consisting of a split/splitless-injector, a head space sampler, column oven and FI-detector. Injection volume: 1 mL of gas from the equilibrated vial (head space). Detector/temperature (250 °C); Hydrogen (40 mL/min); Air (450 mL/min); Make-up gas (N₂: 25 mL/min). **High Performance Liquid Chromatogrrahpy.** An end-capped stainless steel column (125 x 2 mm), filled with Purospher STAR RP18 (5 µM) was used. An acetonitrile/water gradient system was used at a flow rate of 0.3 mL/min and the mobile phase contained 0.05% formic acid. Alternatively, a Spherisorb CN, 5 µm, 250 x4.6 mm column and an isocratic method employing acetonitrile and a pH 5 buffer were employed with a flow rate of 1.0 mL/min. **Titration.** To determine the content of the free base I' in a substance, the substance was dissolved in acetic acid and was titrated potentiometrically with 0.1 N perchloric acid. Equipment: Solvotrode (6.0229.100).

### Materials

4-Benzhydryloxypiperidine **II"** was prepared by a method similar to that previously described (J Med. Chem. 1995, 38, 2472.). 4'-tert-butyl-4-chloro-butyrophenone, methyl isobutyl ketone, sodium hydrogen carbonate, potassium carbonate, potassium hydroxide and sodium hydroxide, *p*-toluenesulfonic acid monohydrate, oxalic acid and phosphoric acid were obtained from commercial chemical suppliers. Phosphoric acid and oxalic acid were titrated before use. All other chemicals were used as received.

### Examples

Examples 1 to 3 show basic experiments. The further examples illustrate preferred embodiments, including the utility of purification procedures for enhancing yield and HPLC purity of I" and/or for the removal of coloured impurities. Some examples illustrate various applicable conditions that can be used for salt formation and neutralisation with the salts disclosed herein.

### Example 1. Synthesis of 4-Benzhydryloxypiperidine Free Base

A solution of 4-hydroxypiperidine (54.83 g, 0.54 mol) and p-toluenesulfonic acid (112.82 g, 0.594 mol) in toluene (216 mL) was heated to reflux (110 °C) and water was removed by azeotropic distillation. A stock solution of benzhydrol (149.23 g, 0.81 mol) in toluene (500 mL) was prepared by heating the mixture of benzhydrol and toluene to 40 °C. A 126 mL portion of the benzhydrol solution was added dropwise to the 4-hydroxypiperidine solution at reflux and the reaction mixture was stirred at reflux for 30 minutes. Then, a second 126 mL portion of benzhydrol solution was added. Further 126 mL portions of the stock solution were added at 30 minute intervals until all of the benzhydrol had been added, azeotropic removal of water was evident during this process. The reaction mixture was stirred at reflux for a further 2 h and then cooled to room temperature. An aqueous solution of sodium hydroxide (2 N, 460 mL, 0.92 mol) was added dropwise with stirring and cooling to the reaction mixture and the phases were separated. The organic phase was washed with water (3x 150 mL, 1 x 170 mL) and then water (570 mL) and acetic acid (38.88 g) were added to the organic phase to extract the 4-benzhydryloxypiperidine. The aqueous solution of 4-benzhydryloxypiperidine acetate was extracted with toluene (3 x 60 mL) and toluene (290 mL) and concentrated sodium hydroxide (67.75 g, 18.75 N) were added. The organic phase containing the product was extracted with water (180 mL and 2 x 150 mL) to give 4-benzhydryloxypiperidine as a toluene solution. The solution was concentrated to give 4-benzhydryloxypiperidine (124.71 g, 86.4 %), as a yellow oil.

### Example 2. Synthesis of 1-[4-(1,1-dimethylethyl)phenyl]-4-[4-(diphenylmethoxy)-1-piperidinyl]-1-butanone I" via benzhydryloxypiperidine tosylate

### 2.1 Synthesis of Benzhydryloxypiperidine toluenesulfonate

Hydroxypiperidine (10.18 g) and p-toluenesulfonic acid (20.92 g) were suspended in toluene (40 mL) and the mixture was heated to reflux (110 °C) to produce a solution. The solution was heated at reflux for 75 minutes during which water was removed by azeotropic distillation. A warm (40 °C) solution of benzhydrol (27.64 g) in toluene (91.4 mL) was added dropwise to the refluxing solution (110-112 °C) in five portions with a 30 minute interval between each addition to allow for azeotropic removal of water. The solution was stirred at reflux for 500 minutes and then cooled to room temperature. The white precipitate was isolated by filtration and washed with toluene (3x20 mL) to give the moist product (41.26 g). The product was dried to give the tosylate (40.81 g). HPLC: 46.31%, II", 53.27% Toluenesulfonic acid. ¹H NMR (300 MHz, DMSO): 8.47-8.40 (2H, m, NH₂), 7.52 (2H, d, J=9 Hz, 2xCH Tosylate), 7.39-7.24 (10H, m, 2 x phenyl), 7.14 (2H, d, J=9 Hz, 2 x CH Tosylate), 5.66 (1H, s, CHO), 3.65-3.60 (1H, m, CHO), 3.17-2.97 (4H, m, CHN), 2.29 (3H, s, CH₃), 2.00-1.93 (2H, m), 1.80-1.73 (2H, m). ¹³C NMR (75 MHz, DMSO): 144.85, 142.62, 137.96, 128.22, 127.15, 126.49, 125.39, 79.21, 68.54, 40.61, 27.52, 20.57.

The obtained II" toluene sulfonate gave the following XRPD Data (intensity values are indicated for illustrative purposes only):

| **Angle** | **d value** | **Intensity** |
|---|---|---|
| **2-Theta°** | **Angstrom** | % |
| 5,881 | 15,01582 | 19,2 |
| 6,835 | 12,92128 | 6,0 |
| 8,280 | 10,66999 | 1,6 |
| 9,679 | 9,13086 | 0,8 |
| 10,643 | 8,30555 | 1,4 |
| 11,784 | 7,50417 | 100,0 |
| 12,669 | 6,98175 | 9,6 |
| 14,050 | 6,29820 | 2,5 |
| 14,524 | 6,09390 | 3,5 |
| 16,227 | 5,45786 | 4,8 |
| 16,761 | 5,28530 | 18,3 |
| 17,738 | 4,99633 | 31,3 |
| 18,020 | 4,91869 | 1,6 |
| 18,740 | 4,73129 | 6,2 |
| 19,185 | 4,62262 | 9,8 |
| 19,492 | 4,55039 | 39,6 |
| 20,007 | 4,43454 | 9,1 |
| 20,667 | 4,29424 | 17,9 |
| 21,023 | 4,22232 | 9,7 |
| 22,175 | 4,00563 | 15,8 |
| 22,391 | 3,96747 | 3,7 |
| 22,630 | 3,92597 | 5,6 |
| 23,020 | 3,86045 | 8,8 |
| 23,363 | 3,80454 | 3,5 |
| 23,721 | 3,74786 | 39,7 |
| 24,065 | 3,69504 | 9,5 |
| 25,164 | 3,53610 | 10,0 |
| 25,750 | 3,45704 | 56,5 |
| 26,724 | 3,33310 | 3,0 |
| 27,122 | 3,28509 | 2,3 |
| 27,423 | 3,24974 | 5,3 |
| 28,001 | 3,18402 | 1,7 |
| 28,380 | 3,14231 | 2,0 |
| 28,615 | 3,11703 | 5,8 |
| 29,357 | 3,03995 | 9,2 |
| 29,990 | 2,97722 | 2,3 |
| 30,867 | 2,89456 | 1,7 |
| 31,395 | 2,84709 | 2,1 |
| 31,672 | 2,82280 | 2,5 |
| 32,301 | 2,76926 | 1,5 |
| 32,563 | 2,74753 | 1,9 |
| 33,280 | 2,69002 | 1,8 |
| 33,647 | 2,66148 | 2,8 |
| 34,110 | 2,62644 | 1,9 |
| 35,250 | 2,54405 | 1,7 |
| 35,930 | 2,49743 | 0,9 |
| 36,597 | 2,45344 | 2,3 |
| 37,700 | 2,38415 | 1,5 |
| 38,263 | 2,35034 | 1,4 |
| 38,748 | 2,32204 | 1,4 |
| 39,167 | 2,29817 | 3,3 |

### 2.2 Synthesis of I" from Benzyhydryloxypiperidine toluenesulfonate

Benzhydryloxypiperidine toluenesulfonate (11.44 g, 0.025 mol), 4'-tert-butyl-4-chlorobutyrophenone (10.15 g, 170 mol %), potassium carbonate (8.47 g, 245 mol %) and methyl isobutyl ketone (18.3 g) were placed in a round bottom flask and a further 20 mL of methyl isobutyl ketone were added and the reaction mixture was placed under a nitrogen atmosphere. The mixture was heated to between 115-116°C and vigorous evolution of gas was noted. The reaction mixture was heated at reflux temperature (105-115 °C) for 2 h and 15 minutes and then cooled to 30 °C internal temperature. Water (50 mL) was added and the phases were separated. The organic phase was washed with water (3x 50 mL) and then concentrated under reduced pressure (45 °C, 8 mbar) to give the crude product. The crude material was suspended in ethanol (30 mL, 96% denatured with toluene) and the mixture was heated to reflux (82°C). The resulting solution was stirred at reflux temperature for 15 minutes and then cooled to 30 °C (the solution was seeded with a small quantity of I") and stirred at 30 °C for 30 minutes. The resulting suspension was then cooled to 2-3°C and stirred at this temperature for 1 h. The product was isolated by filtration and washed with ethanol (3 x 5 mL). The moist product was dried in a vacuum oven (45°C) to give pure I" (6.49 g, 55.27%). HPLC purity (area%): 99.27% RRT 0.62 0.38%.

### 2.3 Synthesis of I" via ebastine tosylate

4-Benzhydryloxypiperidine (13.37 g), 4'-tert-butyl-4-chlorobutyrophenone (20.29 g), sodium hydrogen carbonate (10.29 g) and methyl isobutyl ketone (21.39 g) were heated to reflux temperature (112-116°C) for a total of 7 h 55 minutes and then cooled to room temperature at which point I" of 89.21 % HPLC purity was obtained. Water (100 mL) was added, as was toluene (10 mL). The phases were separated and the organic phase was diluted with toluene (10 mL) and washed with water (100 mL) and this process was repeated twice. Then, the organic phase was concentrated under reduced pressure to afford a distillation residue. The residue was suspended in methyl isobutyl ketone (20 mL) and toluenesulfonic acid monohydrate (11.55 g) was added, and the reaction mixture was warmed to approximately 60°C. Additional methyl isobutyl ketone (50 mL) was added to improve stirring and to dissolve the suspended salt. The super-saturated solution was left to cool and a precipitate formed. The precipitate was isolated by filtration and washed with water (2x25 mL) and methyl isobutyl ketone (2x25mL). The moist product (31.95 g) was dried to give I" tosylate (22.95 g, 64.94%). The tosylate (22.72 g) was suspended in ethanol (228 mL) and potassium carbonate (5.24 g) was added. The mixture was heated to reflux temperature at which point a clear solution had been formed. Water was added (228 mL) and the solution was cooled to 15°C and stirred that this temperature for 45 minutes. The product (18.76 g) was isolated by filtration and dried in an oven at 45 °C to afford pure I" (16.23 g, 69.09%, HPLC purity 99.99%).

### Example 3. Preparation of discoloured 1-[4-(1,1-dimethylethyl)phenyl]-4-[4-(diphenylmethoxy)-1-piperidinyl]-1-butanone I"

A toluene solution of 4-benzhydryloxypiperidine (73.27 g of the toluene solution contained 26.74 g of 4-benzhydryloxypiperidine), sodium hydrogen carbonate (14.28 g), DMF (5.3 mL) and 4'-tert-butyl-4-chloro-butyrophenone (28.65 g) were heated to reflux temperature. The reaction mixture was heated at reflux temperature for 12 h, to produce a solution of I" of 84.3% purity on the basis of HPLC area-% and then cooled to room temperature. The orange reaction mixture was washed with water (3 x 100 mL) and the organic phase was concentrated under reduced pressure. Ethanol (110 mL) was added to the residue and the mixture was heated to reflux. The hot solution was filtered and the filtrate was cooled to 2 °C. The product was isolated by filtration and washed with ethanol (5 x 20 mL). The product was dried in a vacuum oven for several hours to afford the title compound (31.2 g, 66.43 %) as a beige solid (commercial I" is white and specifications (such as those found in the European Pharmacopoeia) require a white to almost white crystalline substance). HPLC purity 99.77 %, significant impurities at relative retention times 0.05 and 0.19 were observed and had HPLC area-% of 0.04 and 0.06%. Discolouration was also sometimes observed when other solvents, including methyl isobutyl ketone were used, but was able to be efficiently suppressed when air was excluded from the reaction mixture, or was able to be efficiently suppressed when an adequate purification procedure by salt formation/neutralization, alternatively or optionally in combination with recrystallization, was used.

### Example 4. Preparation of 1-[4-(1,1-dimethylethyl)phenyl]-4-[4-(diphenylmethoxy)-1-piperidinyl]-1-butanone I"

A mixture of 4-Benzhydryloxypiperidine **II"** (13.37 g, 50 mmol), sodium hydrogen carbonate (10.08 g), methyl isobutyl ketone (21.78 g) and 4'-tert-butyl-4-chloro-butyrophenone III (14.91 g) was heated to reflux (121 °C internal temperature), after 9 h at reflux temperature, HPLC indicated that the solution contained I" of 88.65 % purity and that residual 4-benzhydryloxypiperidine was present to an extent of 2.15 area-%. The mixture was cooled to room temperature. Water (100 mL), methyl isobutyl ketone (25 mL) and toluene (25 mL) were added and the phases were separated. The organic phase was washed with water (2 x 100 mL) and oxalic acid dihydrate (4.45 g) was added. The salt **I"**.H₂C₂O₄ was isolated by filtration and washed with acetone (15 mL). An analytical sample of the oxalate was dried in a vacuum oven and submitted for analysis (HPLC purity: 91.99 area-%). The remaining oxalate was suspended in de-ionized water (100 mL) and stirred. Sodium hydroxide (2N) was gradually added until a pH of 7-7.5 was obtained (approximately 20 mL of base were added). The reaction mixture was stirred at room temperature for 2 h and the resulting 1-[4-(1,1-dimethylethyl)phenyl]-4-[4-(diphenylmethoxy)-1-piperidinyl]-1-butanone **I"** (13.67 g) was isolated by filtration. The moist product was dried in a vacuum oven to give pure 1-[4-(1,1-dimethylethyl)phenyl]-4-[4-(diphenylmethoxy)-1-piperidinyl]-1-butanone **I"** (10.68 g, 45.47 %). HPLC purity 96.3 %; DSC: Onset: 81.63 °C, peak: 84.69 °C, 99.21 J/g, heating rate: 10 °C/min; ¹H NMR (300 MHz, DMSO) 7.88 (d, 2H, ³J = 9 Hz, Ar *CH*=CtBu), 7.51 (d, 2H, ³J = 9 Hz, Ar COC=C*H*), 7.36-7.18 (m, 10H, Ar H), 5.59 (s, 1H, C*H*Ph₂), 3.32-3.27 (m, 1H, C*H*O), 2.93 (t, 2H, ³J = 6 Hz, CH₂N), 2.61 (t, 2H, ³J = 6 Hz, 2xCHN), 2.25 (t, 2H, ³J = 6 Hz, C*H*₂CO), 1.95-1.92 (m, 2H, 2xCHN), 1.77-1.72 (m, 4H, 2xC*H*CHO, CH2), 1.44-1.39 (m, 2H,2x C*H*CHO), 1.29 (s, 9H, 3xCH₃); ¹³C NMR (75 MHz, DMSO): 199.15 (*C*O), 155.68 (Ar *C*CO), 143.04 (2xAr C ipso C on CHPh₂), 134.31 (Ar CtBu), 125.19 (2xAr C*H*=CtBu), 128.02 (Ar CH), 127.83 (Ar CH), 126.68 (Ar CH), 126.43 (Ar CH), 78.76 (CHPh₂), 72.22 (*C*HO), 50.46 (2x*C*H₂N), 35.42 (C*t*Bu), 34.57 (*CH₂*N)*,* 30.97 (*C*H₂), 30.65 (3x*CH₃*), 21.63 (*CH₂*CH₂CO).

### Example 5. Preparation of 1-[4-(1,1-dimethylethyl)phenyl]-4-[4-(diphenylmethoxy)-1-piperidinyl]-1-butanone I"

A mixture of 4-Benzhydryloxypiperidine hydrochloride **II"**.HCl (15.19 g, 50 mmol), sodium hydrogen carbonate (10.08 g), methyl isobutyl ketone (24.30 g) and 4'-tert-butyl-4-chloro*butyrophenone **III"** (14.33 g) was heated to reflux (119 °C) for 11 h (at this point, analysis indicated that an Ebastine solution of 81.94% purity had been formed, based on HPLC area-%). The reaction mixture was cooled to room temperature and water (100 mL) and sodium chloride (1 g) were added. The organic phase was washed with sodium chloride solution (2 x 100 mL) and oxalic acid (4.17 g) was added to the organic phase, the oxalate salt was isolated by filtration and washed twice with acetone to give the moist oxalate salt **I"**.H₂C₂O₄ (22.06 g). The oxalate salt was suspended in water (100 mL) and aqueous ammonia (12.5 % was added dropwise until a pH of 7-7.5 was obtained). The product was isolated by filtration and washed with water (3x 20 mL). The moist product (18.97 g) was dried to give 1-[4-(1,1-dimethylethyl)phenyl]-4-[4-(diphenylmethoxy)-1-piperidinyl]-1-butanone **I"** (13.42 g, 57.15%). HPLC 89.2 %.

### Example 6. Preparation of 1-[4-(1,1-dimethylethyl)phenyl]-4-[4-(diphenylmethoxy)-1-piperidinyl]-1-butanone oxalate I".H₂C₂O₄ (Ebastine Oxalate)

1-[4-(1,1-dimethylethyl)phenyl]-4-[4-(diphenylmethoxy)-1-piperidinyl]-1-butanone **I"** (5.00 g, 10.66 mmol, batch DP023-Su040a, a light pink solid) was dissolved in acetone (25 mL) and oxalic acid (0.96 g, 11 mmol) was added. Precipitation of a salt was evident during the addition and a slight exotherm was noted (the internal temperature rose from 24 to 26 °C). The crude salt was isolated by filtration and was washed with 7 mL of acetone and then with two ice-cold 10 mL portions of acetone. The moist product (9.17 g) was dried in a drying oven at 60 °C to afford the oxalate salt **I"**.H₂C₂O₄ (4.7 g, 84.08 %). HPLC 97.09 %; ¹H NMR (300 MHz, DMSO-D6) δ: 7.54 (2H, d, J= 6, Ar CH tBu), 7.39 (2H, d, J=6, Ar CH), 7.37-7.24 (10H, m, Ar), 5.59 (1H, s, CHPh₂), 4.60-4.50 (2H, m, OH, NH), 3.61-3.59 (1H, m, CHO), 3.22-2.96 (8H, m), 1.98-1.83 (6H, m), 1.30 (9H, s, CH₃x3); ¹³C NMR (75 MHz, DMSO-D6) δ: 198.15 (CO), 164.20 (CO oxalate), 156.15 (Ar CCO), 142.59 (Ar), 133.81 (Ar), 128.19 (Ar), 127.71 (Ar), 127.12, 126.47, 125.32, 79.25, 55.08, 48.75, 34.81, 34.68, 30.67 (3xCH₃), 28.01, 18.41.

### Example 7. Preparation of 1-[4-(1,1-dimethylethyl)phenyl]-4-[4-(diphenylmethoxy)-1-piperidinyl]-1-butanone phosphate I".H₃PO₄

1-[4-(1,1-Dimethylethyl)phenyl]-4-[4-(diphenylmethoxy)-1-piperidinyl]-1-butanone **I"** (14.98 g, 0.032 mol, HPLC purity: 99.79%, major impurities at relative retention times 0.05 and 0.22 were found with areas of 0.07 and 0.04%) was dissolved in acetone (105 mL) and phosphoric acid (3.85 g) was added. After several minutes, the salt precipitated and was isolated by filtration. The salt was washed with acetone (2 x 25 mL) to give the moist product **I"**.H₃PO₄ (22.8 g). HPLC purity 99.98 %; Titration: 93.3 % (NaOH); Mpt 123.7-124.2 °C; ¹H NMR (DMSO-D6) δ: 7.88 (2H, d, J=8.4 Hz), 7.51 (2H, d, J=8.4 Hz), 7.52-7.20 (10H, m), 5.61 (1H, s, CHPh₂), 4.01 (br s, OH (phosphoric acid, water), 3.00 (2H, t, J=6Hz), 2.90-2.86 (2H, m, CHN), 2.59 (2H, t, J=7.2 Hz), 1.87-1.82 (4H, m), 1.65-1.61 (2H, m), 1.28 (9H, s, 3xCH₃). Signals at 3.44, 1.04 correspond to ethanol; ¹³C NMR (DMSO-D6) δ: 199.90 (CO), 156.04 (Ar), 142.95 (Ar), 134.20 (Ar), 128.22 (Ar), 127.80 (Ar), 127.12 (Ar), 126.56 (Ar), 125.38 (Ar), 79.12 (CHO), 55.99 (EtOH), 49.62, 35.31, 34.74, 30.77 (CH₃), 29.56, 20.14, 18.48 (EtOH).

### Example 8. Recrystallization of 1-[4-(1,1-dimethylethyl)phenyl]-4-[4-(diphenylmethoxy)-1-piperidinyl]-1-butanone phosphate I".H₃PO₄

1-[4-(1,1-Dimethylethyl)phenyl]-4-[4-(diphenylmethoxy)-1-piperidinyl]-1-butanone phosphate I".H₃PO₄ (1.0 g, 92.6% (determined by titration with sodium hydroxide)) was suspended in ethanol and the resulting solution was heated to 78 °C. The reaction mixture was stirred at reflux for 10 minutes and allowed to cool slowly to room temperature. At about 55 °C, product precipitation was observed and the suspension was cooled to 2 °C. The product was isolated by filtration and washed with ethanol (2 x 25 mL). The moist product was dried to give 1-[4-(1,1-dimethylethyl)phenyl]-4-[4-(diphenylmethoxy)-1-piperidinyl]-1-butanone phosphate (0.95 g, 95 %). HPLC purity: 99.83 %.

### Example 9. Preparation of 1-[4-(1,1-dimethylethyl)phenyl]-4-[4-(diphenylmethoxy)-1-piperidinyl]-1-butanone I", purification by precipitation of a phosphate salt from the reaction mixture.

A mixture of benzhydryloxypiperidine **II** (13.38 g, 0.05 mol), methyl isobutyl ketone (21.38 g), sodium hydrogen carbonate (10.29 g) and 4'-tert-butyl-4-chloro-butyrophenone **III"** (20.31 g) was heated to reflux (120 °C) and gas evolution was observed. After 4 h 10 minutes, HPLC analysis indicated that a solution of I" of 84.11 % purity, had been formed. The reaction mixture was cooled to room temperature, the organic layer was diluted with toluene (25 mL) and acetic acid 1 % (100 mL) was added. The phases were separated and the organic phase was washed with water (100 mL) and with brine (2 g sodium chloride in 100 mL water). Phosphoric acid (15.52 g, 84.8 %) was added and the saturated solution was seeded with a small quantity of ebastine phosphate **I"**.H₃PO₄ and stored in a refrigerator overnight. The solution was once again seeded with a small quantity of ebastine phosphate and left for a further 3 days in the refrigerator, after which the precipitate was isolated by filtration and washed with cold methyl isobutyl ketone (3x10 mL). The moist product was dried to give Ebastine phosphate I".H₃PO₄ (17.3 g). HPLC purity: 99.48 %.

### Example 10. Washing with acetic acid.

A mixture of 1-[4-(1,1-Dimethylethyl)phenyl]-4-[4-(diphenylmethoxy)-1-piperidinyl]-1-butanone (13.37 g), sodium hydrogen carbonate (10.29 g), methyl isobutyl ketone (21.39 g) and 4'-tert-butyl-4-chlorobutyrophenone (20.29 g) was heated to reflux temperature under a nitrogen atmosphere. After 3 h, HPLC analysis showed that the solution contained Ebastine of 82.60% purity and that the 4-benzhydryloxypiperidine content was 0.58%. The solution was allowed cool to room temperature and 100 g of 2% acetic acid solution was added. The phases were separated and the separatory funnel was washed with toluene (10 mL). The combined organic phases were treated with oxalic acid (11.47 g) and a salt precipitated. Methyl isobutyl ketone (75 mL) was added to facilitate stirring and the salt was isolated by filtration to give 34.98 g of the moist salt. The moist salt was dried in a vacuum drying oven to afford the oxalate (24.13 g). HPLC purity: Ebastine 98.66%, 4-benzhydryloxypiperidine 0.42%, 4'-tert-butyl-4-chlorobutyrophenone 0.47%.

### Example 11. Neutralization of 1-[4-(1,1-dimethylethyl)phenyl]-4-[4-(diphenylmethoxy)-1-piperidinyl]-1-butanone phosphate I".H₃PO₄

1-[4-(1,1-dimethylethyl)phenyl]-4-[4-(diphenylmethoxy)-1-piperidinyl]-1-butanone phosphate **I"**.H₃PO₄ (17.3 g) was dissolved in ethanol (170 mL), sodium hydroxide solution (19.08 g, 6.39 %) was added and the resulting mixture was heated to 78 °C for 15 minutes. The resulting saturated solution was cooled to 35 °C and the resulting free base was cooled to 2-3 °C (in a refrigerator) and isolated by filtration. 1-[4-(1,1-Dimethylethyl)phenyl]-4-[4-(diphenylmethoxy)-1-piperidinyl]-1-butanone **I"** was washed with ethanol (2 x 15 mL) and the resulting moist 1-[4-(1,1-dimethylethyl)phenyl]-4-[4-(diphenylmethoxy)-1-piperidinyl]-1-butanone **I"** (21.22 g) was dried to give the desired product **I"** (16.35 g). HPLC purity: 99.85 %.

### Example 12. Neutralization of 1-[4-(1,1-Dimethylethyl)phenyl]-4-[4-(diphenylmethoxy)-1-piperidinyl]-1-butanone phosphate I".H₃PO₄ in a water/toluene mixture

1-[4-(1,1-Dimethylethyl)phenyl]-4-[4-(diphenylmethoxy)-1-piperidinyl]-1-butanone phosphate **I"**.H₃PO₄ (3.0 g, 5 mmol) was suspended in a mixture of water/toluene (30 mL of a 1:1 mixture) and potassium hydroxide (6.5 g, 10 mmol, 8.85 % solution) was added. After 2 h 10 min, the phases were separated and the aqueous phase was washed with toluene (10 mL). The organic phases were combined and concentrated to give, after drying, 1-[4-(1,1-dimethylethyl)phenyl]-4-[4-(diphenylmethoxy)-1-piperidinyl]-1-butanone **I"** (2.26 g, 91.13 %). HPLC purity: 99.88 %.

### Example 13. Preparation of 1-[4-(1,1-dimethylethyl)phenyl]-4-[4-(diphenylmethoxy)-1-piperidinyl]-1-butanone I"

Ebastine oxalate **I"**.H₂C₂O₄ (3.05 g, 0.005 mol) was suspended in a mixture of toluene (25 mL) and water (25 mL). Potassium carbonate (1.13 g) was added and the suspension was stirred for 20 minutes. Stirring was brought to a halt and the phases were separated. The organic phase was concentrated to give 1-[4-(1,1-dimethylethyl)phenyl]-4-[4-(diphenylmethoxy)-1-piperidinyl]-1-butanone **I"** (2.35 g, 92.97 %). HPLC purity 99.85 %.

### Example 14. Preparation of 1-[4-(1,1-dimethylethyl)phenyl]-4-[4-(diphenylmethoxy)-1-piperidinyl]-1-butanone I"

1-[4-(1,1-Dimethylethyl)phenyl]-4-[4-(diphenylmethoxy)-1-piperidinyl]-1-butanone oxalate **I"**.H₂C₂O₄ (2.93 g, 0.005 mol, HPLC purity: 99.98%, major impurity at relative retention time 0.62: 0.02%) was suspended in a mixture of ethyl acetate (25 mL) and water (25 mL) and potassium carbonate (1.09 g) was added. The phases were separated and the organic phase was concentrated to give a moist product which was dried to give 1-[4-(1,1-dimethylethyl)phenyl]-4-[4-(diphenylmethoxy)-1-piperidinyl]-1-butanone **I"** (2.38 g, 96.75 %). HPLC purity: 99.98 %.

### Example 15.

1-[4-(1,1-Dimethylethyl)phenyl]-4-[4-(diphenylmethoxy)-1-piperidinyl]-1-butanone oxalate **I"**.H₂C₂O₄ (3.58 g, HPLC purity: 99.62%, major impurity at relative retention time 1.08: 0.27%) was suspended in a 1:1 mixture of methyl *tert*-butyl ether and water (50 mL). Aqueous potassium hydroxide (8.11 g, 8.85 %) was added and, after 45 minutes the pH of the reaction mixture was 10. The layers were separated and the organic layer was concentrated under reduced pressure to give 1-[4-(1,1-dimethylethyl)phenyl]-4-[4-(diphenylmethoxy)-1-piperidinyl]-1-butanone (2.90 g). HPLC purity: 99.66 %.

### Example 16.

1-[4-(1,1-Dimethylethyl)phenyl]-4-[4-(diphenylmethoxy)-1-piperidinyl]-1-butanone oxalate **I"**.H₂C₂O₄ (3.58 g) was suspended in MTBE/water (50 mL 1:1) and potassium hydroxide (8.11 g of an 8.85 % solution) was added over 45 minutes until a pH of 10 was obtained. The phases were separated and the aqueous phase was washed with MTBE (10 mL). The combined organic phases were concentrated to give 1-[4-(1,1-dimethylethyl)phenyl]-4-[4-(diphenylmethoxy)-1-piperidinyl]-1-butanone **I"** (2.90 g). HPLC purity: 99.66 %.

### Example 17.

1-[4-(1,1-Dimethylethyl)phenyl]-4-[4-(diphenylmethoxy)-1-piperidinyl]-1-butanone oxalate **I"**.H₂C₂O₄ (3.08 g, HPLC purity: 96.83%) was suspended in a mixture of dichloromethane (25 mL) and water (25 mL) and aqueous sodium hydroxide (5,96 g, 7.39 % solution) was added dropwise with stirring. After 45 minutes, all of the solid material was dissolved and two distinct phases were formed when the mixture was left to stand. The phases were separated and the aqueous phase was extracted with 25 mL of dichloromethane. The combined dichloromethane phases were evaporated to dryness to give 1-[4-(1,1-Dimethylethyl)phenyl]-4-[4-(diphenylmethoxy)-1-piperidinyl]-1-butanone **I"** (2.07 g, 80.09 %,). HPLC purity: 98.14%.

### Example 18. (1-[4-(1,1-dimethylethyl)phenyl]-4-[4-(diphenylmethoxy)-1-piperidinyl]-1-butanone oxalate I".H₂C₂O₄ synthesis and neutralisation)

Oxalic acid (1.24 g) was added to a suspension of 1-[4-(1,1-dimethylethyl)phenyl]-4-[4-(diphenylmethoxy)-1-piperidinyl]-1-butanone **I"** (7.0 g, HPLC purity: 99.66%, major impurity at relative retention time 0.05: 0.08%) in acetone (49 mL). Immediate precipitation of 1-[4-(1,1-dimethylethyl)phenyl]-4-[4-(diphenylmethoxy)-1-piperidinyl]-1-butanone oxalate **I"**.H₂C₂O₄ was observed. The salt was isolated by filtration and washed with acetone. The moist salt was dried in a vacuum drying oven at 45 °C and 36 mbar for 65 minutes to give 1-[4-(1,1-dimethylethyl)phenyl]-4-[4-(diphenylmethoxy)-1-piperidinyl]-1-butanone oxalate **I"**.H₂C₂O₄ (7.98 g, HPLC purity 99.62 %). The salt (4.0 g) was suspended in a mixture of de-ionized water (25 mL) and toluene (25 mL) and stirred. Ammonia solution (1.99 g of an 11.6 % aqueous solution) was added dropwise with stirring and the pH reached 8.5. The reaction mixture was diluted with toluene (100 mL) and de-ionized water (100 mL) and the phases were separated. The organic phase was concentrated under reduced pressure to give 1-[4-(1,1-dimethylethyl)phenyl]-4-[4-(diphenylmethoxy)-1-piperidinyl]-1-butanone **I"** (3.36 g). HPLC purity: 99.36 %.

### Example 19.

Benzhydryloxypiperidine **II**" (13.37 g, 0.05 mol), sodium hydrogen carbonate (10.08 g), methyl isobutyl ketone (32.09 g) and 4-tert-butyl-4-chloro-butyrophenone **III"** (14.33 g) were heated at reflux for 9h 50 min, cooled to room temperature and water (100 mL), toluene (25 mL), and methyl isobutyl ketone (25 mL) were added, at this stage HPLC analysis showed that a solution, which contained **I"** of 87.4 % purity had been formed. The phases were separated and the organic phase was washed with water (2 x 100 mL). Oxalic acid (4.37 g) was added and the product gradually precipitated until stirring was difficult. The moist product **I"**.H₂C₂O₄ (34.99 g, 91.4% HPLC purity) was isolated by filtration and washed with acetone (3 x 15 mL). Water (100 mL) was added to the oxalate salt and then ammonia 12.5% (ca 10 mL) was added until a pH of 7-7.5 was obtained. Moist 1-[4-(1,1-dimethylethyl)phenyl]-4-[4-(diphenylmethoxy)-1-piperidinyl]-1-butanone **I"** (23.67 g) was isolated by filtration and washed with water (4 x 20 mL). The moist product was dried in a vacuum oven to afford the desired product **I"** (18.47 g, 78.53 %, HPLC purity 94.97 %). The dried product (4.90 g) was dissolved in ethanol (10 mL) and heated to reflux. The reaction mixture was cooled to room temperature and the product was isolated by filtration. The product was washed with ethanol and dried to give 1-[4-(1,1-dimethylethyl)phenyl]-4-[4-(diphenylmethoxy)-1-piperidinyl]-1-butanone **I"** (2.76 g, 56.33 % (from the salt)). HPLC purity: 99.50 %.

### Example 20. Preparation of Pure 1-[4-(1,1-dimethylethyl)phenyl]-4-[4-(diphenylmethoxy)-1-piperidinyl]-1-butanone I" from 1-[4-(1,1-dimethylethyl)phenyl]-4-[4-(diphenylmethoxy)-1-piperidinyl]-1-butanone oxalate I".H₂C₂O₄

1-[4-(1,1-Dimethylethyl)phenyl]-4-[4-(diphenylmethoxy)-1-piperidinyl]-1-butanone oxalate **I"**.H₂C₂O₄ (1.10 g, HPLC purity: 97.78%, major impurity relative retention time 0.03: 1.60%) was added with stirring to a solution of NaOH (0.16 g) in ethanol (15 mL) at 60 °C. Then, the solution was cooled to 4 °C in a refrigerator before isolation of Ebastine by filtration. The resulting 1-[4-(1,1-dimethylethyl)phenyl]-4-[4-(diphenylmethoxy)-1-piperidinyl]-1-butanone **I"** was washed with water (2 x 5 mL) and dried at 60 °C for 6 h to afford pure 1-[4-(1,1-dimethylethyl)phenyl]-4-[4-(diphenylmethoxy)-1-piperidinyl]-1-butanone **I"** (0.53 g, HPLC purity 97.94%, major impurity at relative retention time 0.03:1.63% ).

### Example 21. Preparation of 1-[4-(1,1-Dimethylethyl)phenyl]-4-[4-(diphenylmethoxy)-1-piperidinyl]-1-butanone Tosylate I".HOSO₃Tol

1-[4-(1,1-Dimethylethyl)phenyl]-4-[4-(diphenylmethoxy)-1-piperidinyl]-1-butanone **I"** (1.01 g, HPLC purity: 99.94%, major impurity, relative retention time 1.43: 0.04%) was dissolved in a mixture of methyl isobutyl ketone (5 mL) and ethanol (5 mL, 96 % denatured with toluene) and toluenesulfonic acid monohydrate (0.41 g) was added. The salt precipitated gradually over 5 minutes then, the product was isolated by filtration to give the moist product (1.37 g). Moist 1-[4-(1,1-dimethylethyl)phenyl]-4-[4-(diphenylmethoxy)-1-piperidinyl]-1-butanone **I"** was dried under vacuum to give the desired compound **I"** as a white solid (0.96 g, 67.61%). HPLC: 1-[4-(1,1-dimethylethyl)phenyl]-4-[4-(diphenylmethoxy)-1-piperidinyl]-1-butanone: 54.57%, p-Toluenesulfonate: 44.99%, benzhydrol: 0.45 %. ¹H NMR (300 MHz, DMSO-D6) δ: 9.20-9.04 (br s, 1H, NH⁺), 7.89 (d, ³J = 9 Hz, 2H, 2x *CH*CHCSO₃), 7.53 (d, ³J = 9 Hz, 2H, Arom. 2xCHCHCCO), 7.51 (d, 3J = 9 Hz, 2H, Arom. 2xCHCCO), 7.40-7.24 (10 H, m, arom H.), 7.09 (d, 2H, *CH*CSO₃), 5.67 (s, 1H, *CH*Ph₂), 3.80-2.90 (m, 10H), 2.25 (s, 3H, CH₃), 2.10-1.74 (m, 4H), 1.39 (s, 9H, tBu).

### Example 22. Synthesis of I" from I".HOSO₃Tol

1-[4-(1,1-Dimethylethyl)phenyl]-4-[4-(diphenylmethoxy)-1-piperidinyl]-1-butanone tosylate **I"**.HOSO₃Tol (3.00 g, 4.67 mmol, HPLC purity: I" 52.98 area-%, II": 0.04 area-%, benzhydrol dimer: 0.07 area-%) was suspended in ethanol (30 mL, 96 %, denatured with toluene) and potassium carbonate (0.69 g, 5.14 mmol, 110 mol%) was added. The reaction mixture was stirred at room temperature for 1 h, after which de-ionized water (30 mL) was added. The reaction mixture was stirred for a further 5 minutes at room temperature and filtered to give moist 1-[4-(1,1-dimethylethyl)phenyl]-4-[4-(diphenylmethoxy)-1-piperidinyl]-1-butanone. The moist product was dried in a drying oven to afford dry 1-[4-(1,1-dimethylethyl)phenyl]-4-[4-(diphenylmethoxy)-1-piperidinyl]-1-butanone **I"** (2.09 g, 97.88 %). HPLC purity: 92.63%, 7.12 % (p-toluenesulfonic acid).

### Example 23.

p-Toluenesulfonic acid monohydrate (60.79 g, 0.32 mol) was added to a solution of crude Compound I" (295 mL, containing **I"** of 61.45 area-% purity by HPLC analysis) and the solution was stirred at room temperature for 15 minutes. Then, the tosylate salt **I"**.HOSO₃Tol slowly precipitated. The reaction mixture was cooled to 4 °C and isolated by filtration and the salt was washed with cold ethanol (2 x 10 mL). The product was dried to afford I".HOSO₃Tol (7.27 g). The salt was stirred in ethanol (20 mL) for 10 minutes, isolated by filtration and washed with ethanol (5 mL) and the moist product was dried to give I" (6.35 g). HPLC: toluenesulfonate 46.14 % and compound I" 53.57 %.

### Example 24.

Tosylate salt I" (2.02 g, HPLC purity I": 54.11% toluenesulfonate: 45.89%) was suspended in ethanol (20 mL) and potassium carbonate (0.46 g) was added, the reaction mixture was heated to reflux temperature and then cooled to room temperature over 52 minutes. The product **I"** was isolated by filtration and washed with ethanol (10 mL) and the moist product was dried to afford **I"** (0.62 g). The mother liquor was left to stand overnight and toluenesulfonic acid precipitated. The sulfonic acid (0.59 g) was isolated by filtration and was dried to afford 0.49 g of **I"**. HPLC (I"): 99.9 %.

## Claims

1. A process to prepare a compound of formula **I"** in free base form or in salt form, 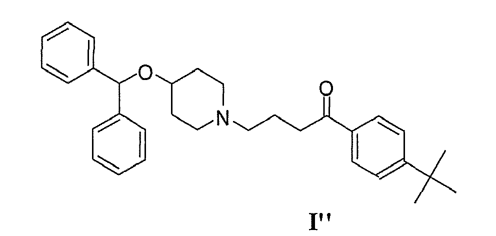 the process comprising:
(i) reacting a compound of formula V"' 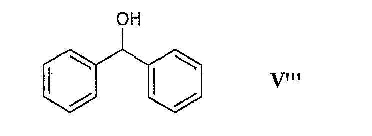 with 4-hydroxypiperidine or an acid addition salt thereof in the presence of a solvent or mixture of solvents to obtain a compound of formula **II"** 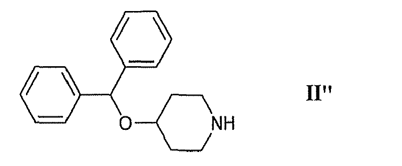 or an acid addition salt thereof,
(ii) subsequently reacting the compound of formula **II"** or the acid addition salt thereof with a compound of formula **III"'** 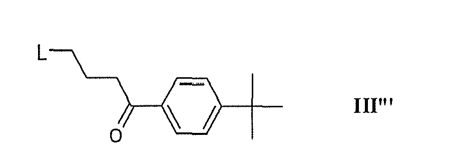 wherein L represents a leaving group, in the presence of a solvent or mixture of solvents, preferably in the presence of an auxiliary base, to produce a compound of formula **I"** or an acid addition salt thereof; and
(iii) isolating and optionally recrystallizing the free base of the compound of formula **I"**,
and/or
(iii') precipitating or crystallizing an acid addition salt of the compound of formula **I"** produced in step (ii) by contact with an acid in the presence of a suitable solvent or mixture of solvents, optionally isolating the acid addition salt of the compound of formula **I",** and converting the acid addition salt of the compound of formula **I"** into the free base of a compound of formula **I"**.

2. The process according to claim 1, wherein any one of said acid addition salts are independently chosen to be respectively soluble in hot solvent solution, but are subject to precipitation upon decreasing the temperature, preferably at about 20-30 °C or below.

3. The process according to any one of the preceding claims, wherein step (ii) comprises:
**a')** providing respectively a compound of formula **II"** or an acid addition salt thereof, optionally dissolved in a first solvent, preferably adding an auxiliary base and/or a second solvent, and a compound of formula **III"'**,
**b')** heating the mixture obtained in step a'), preferably to reflux temperature, even more preferably under agitation until complete conversion is observed,
**c')** optionally and preferably allowing the mixture to cool, more preferably to ambient temperature, and preferably diluting the mixture with a third solvent,
**d')** adding water, optionally an auxiliary substance selected from sodium chloride and a proton donor, preferably as an aqueous solution, more preferably under agitation and separating the phases,
**e')** optionally repeating the procedure as described in step **d')**, more preferably repeating said procedure twice,
**f')** adding a proton donor to the organic phase obtained in step d') or optional step e'), optionally seeding the mixture in order to induce crystallisation, and allowing an acid addition salt of a compound of formula **I"** to precipitate.

4. The process according to any one of the preceding claims, wherein in step (iii)/(iii') the free base of the compound of formula **I"** is isolated and converted into the acid addition salt of the compound of formula **I"** comprising the steps of:
**g')** concentrating the organic phase obtained in step d') or optionally e'), preferably under reduced pressure, more preferably at reflux temperature, and optionally adding an appropriate solvent and heating the mixture,
**h')** optionally removing insolubles, preferably by filtration, cooling, and allowing the crude free base of a compound of formula **I"** to precipitate,
**i')** isolating, preferably washing, and optionally drying the solid obtained in step h')
**j')** optionally re-crystallizing the solid obtained in step i') from an appropriate solvent and optionally drying the solid,
**k')** and dissolving the solid obtained in step i') or j') respectively in an appropriate solvent, adding a proton donor, optionally seeding the mixture in order to induce crystallisation, and allowing an acid addition salt of a compound of formula **I"** to precipitate.

5. The process according to any one of the preceding claims, wherein the acid addition salt of a compound of formula **I"** is re-crystallised comprising the steps of:
**l')** providing the acid addition salt of a compound of formula **I"**, and adding an appropriate solvent or mixture of solvents and preferably heating the mixture, more preferably to reflux temperature,
**m')** optionally filtering the mixture, and allowing the solution to cool, optionally seeding the mixture in order to induce crystallisation, and
**n')** allowing said acid addition salt of a compound of formula **I"** to precipitate or crystallize.

6. Use of an acid addition salt of benzhydroxylpiperidine of formula II" 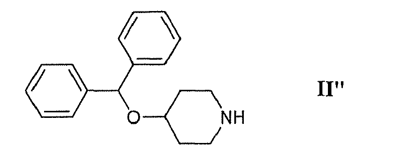 for preparing a crystalline form of the free base compound of formula **I"**, wherein said acid addition salt is selected from an oxalate salt, a toluenesulfonate salt and a phosphate salt, preferably the toluenesulfonate salt:

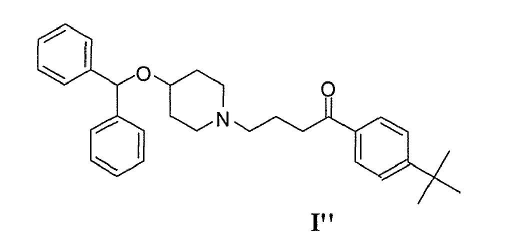

7. An acid addition salt of benzhydroxylpiperidine of formula **II"** 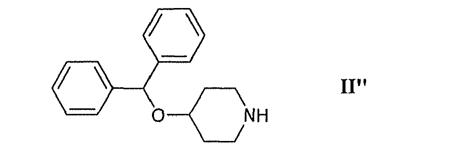 which acid addition salt is selected from an oxalate salt, a toluenesulfonate salt and a phosphate salt, and is preferably the toluenesulfonate salt.

8. Process for preparing a crystalline form of the free base compound of formula **I",** 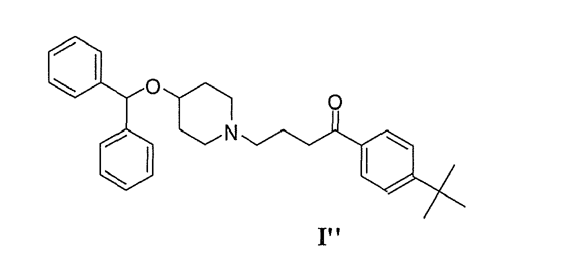 wherein an acid addition salt of the compound of formula **I"** is reacted with a proton acceptor in the presence of at least one suitable solvent, wherein said acid addition salts is selected from an oxalate salt, a toluenesulfonate salt and a phosphate salt, and then allowing said free base compound of formula **I"** to precipitate or crystallize.

9. The process according to any one of the preceding claims, wherein the finally obtained free base of a compound of formula **I"** is **characterized by** an impurity profile of not more than 0.10 % of each individual impurity and gives a colourless, clear dichloromethane solution, and/or wherein the acid addition salt of the compound of formula **I"** has a purity of at least 80 %, preferably not less than 97 %, and even more preferably greater than 99.4 %.

10. Crystalline free base compound of formula **I"** exhibiting an X-ray powder diffraction with at least the following significant signals at the indicated d-values, respectively with a tolerance of ± 0.2 (2-Theta °): 16.1, 16.9, 18.8 and 19.4.

11. A process to prepare a pharmaceutical composition comprising a compound of formula **I"** or a pharmaceutically acceptable salt thereof, comprising the steps of
(i) carrying out a process according to any one of claims 1 to 6, 8 and 9,
(ii) optionally reacting free base of compound of formula **I"** with an organic or inorganic acid of a pharmaceutically acceptable anion to give the corresponding pharmaceutically acceptable salt; and
(iii) mixing said free base of the compound of formula **I"** or a pharmaceutically acceptable salt thereof with a pharmaceutically acceptable excipient.

12. A crystalline phosphate salt of a compound of formula **I"**, preferably exhibiting an X-ray powder diffraction with at least the following significant signals at the indicated d-values, respectively with a tolerance of ± 0.2 (2-Theta °): 7.2, 14.0, 14.5, 19.0 and 20.1.

13. A crystalline oxalate salt of a compound of formula **I"**, preferably exhibiting an X-ray powder diffraction with at least the following significant signals at the indicated d-values, respectively with a tolerance of ± 0.2 (2-Theta °): 16.1, 17.0, 18.8, 19.4 and 31.6.

14. A crystalline toluene sulfonate salt of a compound of formula **I"**, preferably exhibiting an X-ray powder diffraction with at least the following significant signals at the indicated d-values, respectively with a tolerance of ± 0.2 (2-Theta °): 4.3, 19.4, 20.1 and 23.7.

15. Use of an acid addition salt of a compound of formula **I"** selected from the group consisting of phosphate, toluenesulfonate and an oxalate salts as defined in any one of claims 12 to 14 in a process for the manufacture of a pharmaceutically active ingredient, optionally wherein said acid addition salt of a compound of formula **I"** is converted to the free base of the compound of formula **I"** to be used in the pharmaceutical formulation.
